# EUROPEAN PATENT APPLICATION

(11) **EP 4 616 819 A1**
(43) Date of publication of application: **17.09.2025**
(21) Application number: 24188971.6
(22) Date of filing: 16.07.2024
(51) Int. Cl.: A61B 34/10, A61B 8/00, A61B 18/14, A61B 34/00, A61B 90/00, A61B 18/00, A61B 34/20

(54) **MEDICAL IMAGING APPARATUS AND METHOD FOR CONTROLLING MEDICAL IMAGING APPARATUS**

(30) Priority: 11.03.2024 KR 20240034137; 17.05.2024 KR 20240064830
(71) Applicant: Samsung Medison Co., Ltd., Hongcheon-gun, Gangwon-do 25108 (KR)
(72) Inventor: SONG, Minjung, 25108 Nam-myeon, Hongcheon-gun (KR); LEE, Jungwoo, 25108 Nam-myeon, Hongcheon-gun (KR)
(74) Representative: Frey, Sven Holger

(57) **Abstract**

Disclosed is a medical imaging apparatus including a display, and a processor configured to display a medical image on the display, recognize an ablation electrode from the medical image, display an ablation expected area of the recognized ablation electrode on the medical image, and control a size of the ablation expected area based on a set output of the ablation electrode.

## Description

### BACKGROUND

### 1. Field

The disclosure relates to a medical imaging apparatus providing improved user experience and a method for controlling the medical imaging apparatus.

### 2. Description of the Related Art

Recently, various types of medical imaging apparatuses to obtain information on human living tissues by imaging have widely been used in the medical field for the purpose of early diagnosis of and surgery on various diseases. Examples of the medical imaging apparatuses may include ultrasound imaging apparatuses, computed tomography (CT) apparatuses, and magnetic resonance imaging (MRI) apparatuses.

An ultrasound imaging apparatus is an apparatus that noninvasively obtains at least one image of an internal part (e.g., soft tissue or blood flow) of an object by emitting ultrasound signals generated by a transducer of a probe to the object and receiving information of echo signals from the object. Ultrasound imaging apparatuses may be used for medical purposes such as observation of an internal area of an object, detection of foreign substances, and measurement of injury severity. Such ultrasound imaging apparatuses are widely used with other imaging apparatuses due to higher stability than other imaging apparatuses using X-rays, the ability to display images in real time, and safety without exposure to radiation.

A computed tomography (CT) apparatus is a medical imaging apparatus used to observe an internal structure of the body. A CT apparatus uses X-rays to observe various tissues in the body in more detail. CT scanning is used in a wide range of medical fields and assists diagnosis of various diseases or injuries such as tumors, bleeding, and inflammation. In addition, CT scanning provides important information to understand a structure and functions of the body. CT scanning is also useful in emergency situations due to ease of operation and quick results.

The ultrasound imaging apparatus and the CT apparatus may be used with a radiofrequency generator that ablates a lesion by using electrical energy of radiofrequency.

For example, a medical personnel may proceed with an ablation procedure by inserting an ablation electrode of a radiofrequency generator into an object, observing an object to be ablated as a target of ablation by using an ultrasound imaging apparatus, and predicting an ablation area based on skills and experiences thereof.

### SUMMARY

It is an aspect of the disclosure to provide a medical imaging apparatus providing improved user experience while proceeding with an ablation procedure, and a method for controlling the medical imaging apparatus.

Additional aspects of the disclosure will be set forth in part in the description which follows and, in part, will be obvious from the description, or may be learned by practice of the disclosure.

In accordance with an aspect of the disclosure, a medical imaging apparatus includes a display, and a processor configured to display a medical image on the display, recognize an ablation electrode from the medical image, display an ablation expected area of the recognized ablation electrode on the medical image, and control a size of the ablation expected area based on a set output of the ablation electrode.

The processor may increase the size of the ablation expected area as the set output of the ablation electrode increases.

The processor may display a visual indicator corresponding to the ablation expected area, and control a size of the visual indicator based on the set output of the ablation electrode.

The processor may display the ablation expected area as a circular visual indicator and control a radius of the circle based on a set output of the ablation electrode.

Based on an electrical energy corresponding to the set output being applied to the ablation electrode, the processor may display the ablation expected area to be visually distinguished from the other areas in the medical image.

Based on an electrical energy corresponding to the set output being applied to the ablation electrode, the processor may display information on an ablation order of the ablation expected area.

Based on an electrical energy corresponding to the set output being applied to the ablation electrode, the processor displays information on the electrical energy applied to the ablation expected area.

Based on an electrical energy corresponding to the set output being applied to the ablation electrode, the processor displays a visual indicator showing a current flow on the recognized ablation electrode.

The ablation electrode may include a first ablation electrode and a second ablation electrode.

The processor may receive a user input for matching each of the first ablation electrode and the second ablation electrode to each of a plurality of ablation electrodes recognized from the medical image, control a size of a first ablation expected area of a first recognized ablation electrode matched to the first ablation electrode according to the user input based on a first set output of the first ablation electrode, and control a size of a second ablation expected area of a second recognized ablation electrode matched to the second ablation electrode according to the user input based on a second set output of the second ablation electrode.

The ablation electrode may include a positive electrode and a negative electrode.

The processor may receive a user input for matching each of the positive electrode and the negative electrode to each of a plurality of ablation electrodes recognized from the medical image, display the ablation expected area based on a position of the first recognized ablation electrode matched to the positive electrode according to the user input and a position of the second recognized ablation electrode matched to the negative electrode according to the user input, and control a size of the ablation expected area based on the set output of the positive electrode.

The processor may display a guide image visualizing an object to be ablated on the display together with the medical image, and display an ablation completed area on the guide image based on an operation of the ablation electrode.

In accordance with another aspect of the disclosure, a method for controlling a medical imaging apparatus includes displaying a medical image, recognizing the ablation electrode from the medical image, displaying an ablation expected area of the recognized ablation electrode on the medical image, and controlling a size of an ablation expected area based on a set output of the ablation electrode.

The controlling of the size of the ablation expected area may include increasing the size of the ablation expected area as the set output of the ablation electrode increases.

The displaying of the ablation expected area may include displaying a visual indicator corresponding to the ablation expected area.

The controlling of the size of the ablation expected area may include controlling a size of a visual indicator based on the set output to the ablation electrode

The displaying of the ablation expected area may include displaying the ablation expected area with a circular visual indicator.

The controlling of the size of the ablation expected area may include a radius of the circle based on the set output of the ablation electrode.

The method for controlling the medical imaging apparatus may further include displaying the ablation expected area to be visually distinguished from the other areas in the medical image based on an electrical energy corresponding to the set output being applied to the ablation electrode.

The method for controlling the medical imaging apparatus may further include displaying information on an ablation order of the ablation expected area based on an electrical energy corresponding to the set output being applied to the ablation electrode.

The method for controlling the medical imaging apparatus may further include displaying information on an electrical energy applied to the ablation expected area upon application of the electrical energy corresponding to the set output to the ablation electrode.

The method for controlling the medical imaging apparatus may further include displaying a visual indicator showing a current flow on the recognized ablation electrode based on an electrical energy corresponding to the set output being applied to the ablation electrode.

The method for controlling the medical imaging apparatus may further include receiving a user input for matching each of the first ablation electrode and the second ablation electrode to each of a plurality of ablation electrodes recognized from the medical image.

The controlling of the size of the ablation expected area may include controlling a size of a first ablation expected area of a first recognized ablation electrode matched to the first ablation electrode according to the user input based on a first set output of the first ablation electrode, and controlling a size of a second ablation expected area of a second recognized ablation electrode matched to the second ablation electrode according to the user input based on a second set output of the second ablation electrode.

The method for controlling the medical imaging apparatus may further include receiving a user input for matching each of the positive electrode and the negative electrode to each of a plurality of ablation electrodes recognized from the medical image.

The displaying of the ablation expected area may include displaying the ablation expected area based on a position of the first recognized ablation electrode matched to the positive electrode according to the user input and a position of the second recognized ablation electrode matched to the negative electrode according to the user input, and

The controlling of a size of an ablation expected area may include controlling a size of the ablation expected area based on the set output of the positive electrode

The method for controlling the medical imaging apparatus may further include displaying a guide image visualizing an object to be ablated together with the medical image, and displaying an ablation completed area on the guide image based on an operation of the ablation electrode.

### BRIEF DESCRIPTION OF THE DRAWINGS

These and/or other aspects of the disclosure will become apparent and more readily appreciated from the following description of the embodiments, taken in conjunction with the accompanying drawings, wherein like reference numerals denote like structural elements, of which:
FIGS. 1A and 1B are block diagrams illustrating configurations of ultrasound imaging system according to an embodiment of the disclosure;
FIGS. 2A, 2B, 2C, and 2D are diagrams illustrating ultrasound imaging apparatuses according to an embodiment of the disclosure;
FIG. 3 is a diagram illustrating an ablation device according to an embodiment of the disclosure;
FIG. 4 is a block diagram illustrating a configuration of the ablation device according to an embodiment of the disclosure;
FIG. 5 is a diagram illustrating that an ablation procedure is performed on an object by using an ablation electrode and an ultrasound probe;
FIG. 6 is a flowchart illustrating an example of a method for controlling an ultrasound imaging apparatus according to an embodiment of the disclosure;
FIG. 7 illustrates an example of an interface provided by an ultrasound imaging apparatus according to an embodiment of the disclosure;
FIG. 8 is a diagram illustrating a change in the size of an ablation expected area displayed by the ultrasound imaging apparatus according to an embodiment of the disclosure;
FIG. 9 is a diagram illustrating an area where ablation is completed in an ultrasound image and/or in a guide image displayed by an ultrasound imaging apparatus according to an embodiment of the disclosure;
FIG. 10 is a diagram illustrating an example of ablation history information displayed by the ultrasound imaging apparatus according to an embodiment of the disclosure;
FIG. 11 is a diagram illustrating a current flow of an ablation electrode displayed by an ultrasound imaging apparatus according to an embodiment of the disclosure;
FIG. 12 is a flowchart illustrating an example of a method for controlling an ultrasound imaging apparatus according to an embodiment of the disclosure;
FIG. 13 illustrates an example of a method of matching a plurality of ablation electrodes to a plurality of recognized ablation electrodes by using an ultrasound imaging apparatus according to an embodiment of the disclosure;
FIG. 14 illustrates a plurality of ablation expected areas with regard to a plurality of ablation electrodes displayed by an ultrasound imaging apparatus according to an embodiment of the disclosure; and
FIG. 15 is a diagram illustrating one ablation expected area with regard to a plurality of ablation electrodes displayed by an ultrasound imaging apparatus according to an embodiment of the disclosure.

### DETAILED DESCRIPTION

The present specification describes the principles of the disclosure and discloses embodiments such that the scope of the disclosure may be clarified and those skilled in the art to which the disclosure pertains may implement the present invention. The disclosed embodiments may be implemented in various forms.

Throughout the specification, like reference numerals refer to like elements. This specification does not describe all the elements of the embodiments, and general contents or duplicative contents between embodiments in the technical field of the disclosure will be omitted. As used herein, the terms 'module' or 'unit' may be embodied as one of software, hardware, or firmware, or any combination thereof, and it is also possible for a plurality of 'modules' or 'units' to be embodied as one component, or one 'module' or 'unit' to include a plurality of components.

Singular forms of nouns corresponding to items are intended to include one or multiple items as well, unless the context clearly indicates otherwise.

Throughout the present disclosure, expressions such as "A or B", "at least one of A and B", "at least one of A or B", "A, B or C", "at least one of A, B, and C", and "at least one of A, B, or C" may include only one of the listed items of each expression or any combination thereof.

The term "and/or" includes any combinations of a plurality of associated listed components or any one of the plurality of associated listed components.

The terms "first", "second", etc. are used to distinguish one component from other components and are not used to limit the components in other aspects (e.g., importance or order).

Also, the terms such as 'front surface', 'rear surface', 'upper surface', 'lower surface', 'side surface', 'left', 'right', 'upper', and 'lower' are defined based on the drawings and the shape and position of each element are not limited by these terms.

The terms such as "include" and "have" are intended to indicate the existence of features, numbers, processes, operations, components, parts, or combinations thereof disclosed in the specification, and are not intended to preclude the possibility that one or more other features, numbers, processes, operations, components, parts, or combinations thereof may exist or may be added.

When an element is referred to as being "connected to", "coupled to", "supported by", or "in contact with" another element, it may be not only directly connected to, coupled to, supported by, or in contact with the other element but also indirectly connected to, coupled to, supported by, or in contact with the other element via a third intervening element.

It will be understood that when one element, is referred to as being "on" another element, it can be directly on the other element, or intervening elements may also be present therebetween.

Hereinafter, an ultrasound apparatus according to various embodiments will be described in detail with reference to the drawings. In the descriptions with reference to the drawings, like reference numerals refer to like elements and detailed descriptions given above may be omitted.

In the disclosure, the 'medical image' may include a medical image obtained by a medical imaging apparatus such as a magnetic resonance imaging (MRI) apparatus, a computed tomography (CT) apparatus, an ultrasound imaging apparatus, or an X-ray imaging apparatus.

As used herein, the term 'object' refers to a target to be imaged and may include a human, an animal, or a part thereof. For example, the object may include a part of the body (e.g., organ), a phantom, or the like.

As used herein, the term 'ultrasound image' refers to an image of an object generated or processed based on ultrasound signals (echo signals) emitted to the object and reflected thereby.

As used herein, the term 'object to be ablated' refers to an object that is required to receive an ablation procedure or a lesion contained in the object.

As used herein, the term 'object' refers to a certain part (e.g., liver or thyroid) of the body of an animal (e.g., human).

As used herein, the term 'user' may include not only medical professionals (e.g., doctors and sonographers) but also all subjects using a medical imaging apparatus.

As used herein, the term 'visual indicator' may include various indicators such as text, number, figure (e.g., dot, line, plane, and three-dimensional structure), color, animation, and visual effect.

Hereinafter, embodiments of the disclosure will be described with reference to the drawings.

In the disclosure, a 'medical imaging apparatus' may include not only an ultrasound imaging apparatus, which will be described below, but also a CT imaging apparatus.

A CT imaging apparatus may include an X-ray generator and an image processor configured to obtain an X-ray image based on X-ray data by using X-rays passing through an object.

Hereinafter, an ultrasound imaging apparatus will be described as an example of the medical imaging apparatus for descriptive convenience, but all embodiments to be described below may also be applied to a CT imaging apparatus.

For example, an ultrasound probe configured to receive echo signals to generate an ultrasound image in an ultrasound imaging system may correspond to an X-ray emitter configured to emit X-rays and an X-ray detector configured to detect X-rays passing through a patient in a CT imaging system. In addition, an ultrasound image in the ultrasound imaging system may correspond to an X-ray image in a CT imaging system.

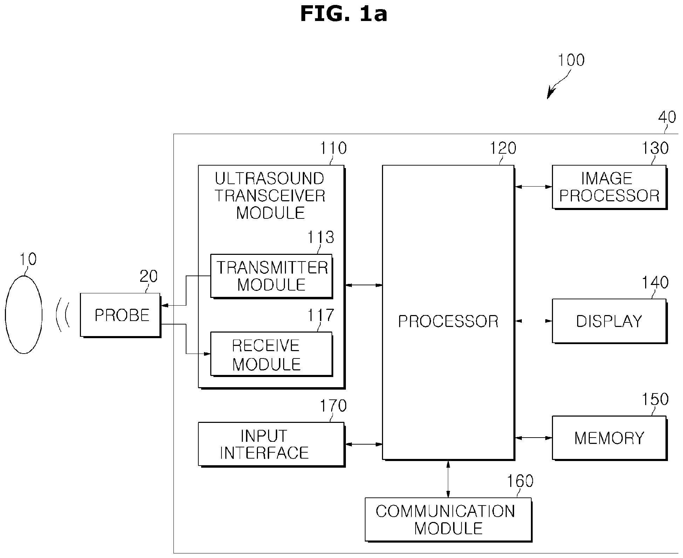

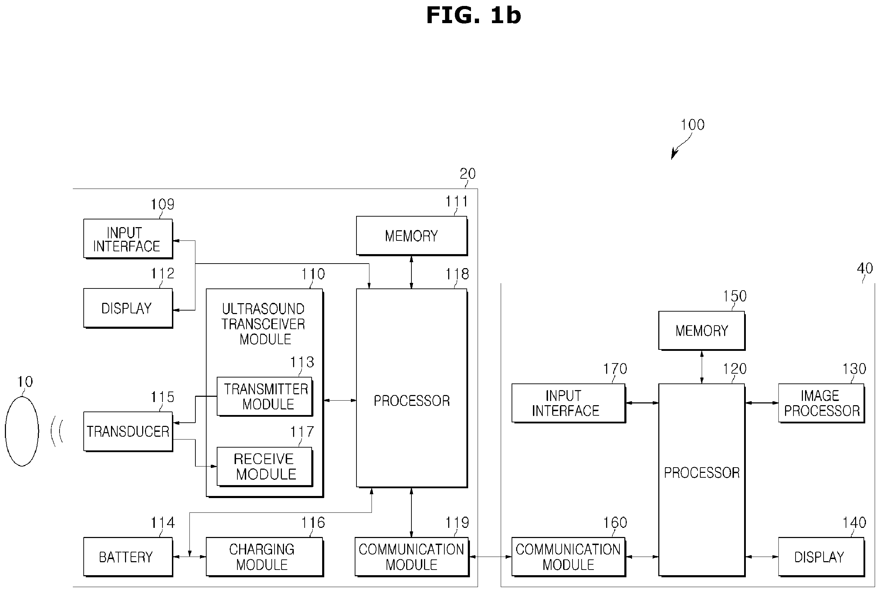

FIGS. 1A and 1B are block diagrams illustrating configurations of ultrasound imaging system according to an embodiment of the disclosure.

According to an embodiment, a medical imaging apparatus may include an ultrasound imaging apparatus 40 and/or a CT imaging apparatus. Hereinafter an ultrasound imaging apparatus 40 will be described, as an medical imaging apparatus, for descriptive convenience.

Referring to FIGS. 1A and 1B, an ultrasound imaging system 100 may include a probe 20 and an ultrasound imaging apparatus 40.

The ultrasound imaging apparatus 40 may be implemented not only as a cart type apparatus but also as a portable apparatus. Examples of the portable ultrasound imaging apparatus may be a smart phone, a laptop computer, a personal digital assistant (PDA), or a tablet personal computer (PC), each including a probe and an application, but are not limited thereto. The ultrasound imaging apparatus 40 may be implemented integrally with the probe.

The probe 20 may include a wired probe connected to the ultrasound imaging apparatus 40 by wire and communicating with the ultrasound imaging apparatus 40 in a wired manner, a wireless probe connected to the ultrasound imaging apparatus 40 wirelessly and communicating with the ultrasound imaging apparatus 40 in a wireless manner, and/or a hybrid probe connected to the ultrasound imaging apparatus 40 by wire or wirelessly and communicating with the ultrasound imaging apparatus 40 in a wired or wireless manner.

According to various embodiments of the disclosure, the ultrasound imaging apparatus 40 may include an ultrasound transceiver module 110 as shown in FIG. 1A, or the probe 20 may include an ultrasound transceiver module 110 as shown in FIG. 1B. According to various embodiments of the disclosure, both the ultrasound imaging apparatus 40 and the probe 20 may also include an ultrasound transceiver module 110.

The probe 20 may also be referred to as an ultrasound probe because the probe transmits and receives ultrasound signals.

According to various embodiments of the disclosure, the probe 20 may further include at least one, or any combination, of an image processor 130, a display 140, or an input interface 170. In the disclosure, descriptions regarding the ultrasound transceiver module 110, the image processor 130, the display 140, or the input interface 170 included in the ultrasound imaging apparatus 40 may also be applied to the ultrasound transceiver module 110, the image processor 130, the display 140, or the input interface 170 included in the probe 20.

FIG. 1A is a block diagram illustrating a configuration of the ultrasound imaging system 100 in the case where the probe 20 is a wired probe or a hybrid probe.

The probe 20 may include a plurality of transducers. The plurality of transducers may be arranged in a predetermined arrangement to be implemented as a transducer array. The transducer array may correspond to a one-dimensional (1D) array or a two-dimensional (2D) array. The plurality of transducers may transmit ultrasound signals to an object 10 in accordance with a transmission signal received from a transmitter module 113. The plurality of transducers may generate a reception signal by receiving ultrasound signals (echo signals) reflected by the object 10. In addition, the probe 20 may be implemented integrally with the ultrasound imaging apparatus 40 or implemented as a separate type connected to the ultrasound imaging apparatus 40 by wire. In addition, the ultrasound imaging apparatus 40 may be connected to one or a plurality of probes 20 depending on the type of implementation.

In the case of a wired probe or a hybrid probe, the probe 20 may include a cable or a connector connectable to a connector of the ultrasound imaging apparatus 40.

The probe 20 according to an embodiment of the disclosure may be implemented as a 2D probe. In the case where the probe 20 is implemented as a 2D probe, the plurality of transducers included in the probe 20 may be arranged two-dimensionally to constitute a 2D transducer array.

For example, the 2D transducer array may include a plurality of sub-arrays, each including a plurality of transducers arranged in a first direction, in a second direction different from the first direction.

In addition, in the case where the probe 20 according to an embodiment of the disclosure is implemented as a 2D probe, the ultrasound transceiver module 110 may include at least one of an analog beamformer or a digital beamformer. In addition, the 2D probe according to an embodiment of the disclosure may include at least one, or any combination, of an analog beamformer or a digital beamformer depending on the type of implementation.

The processor 120 controls the transmitter module 113 to form the transmission signal to be applied to each of the transducers 115 in consideration of positions and focal points of the plurality of transducers included in the probe 20.

The processor 120 may control the receiver module 117 to generate ultrasound data by performing analog-to-digital conversion on reception signals received from the probe 20, and combining digital-converted reception signals in consideration of positions and focal points of the plurality of transducers.

In the case where the probe 20 is implemented as a 2D probe, the processor 120 may calculate a time delay value for digital beamforming of each of the plurality of sub-arrays included in the 2D transducer array on the basis of the sub-array. In addition, the processor 120 may calculate a time delay value for analog beamforming of each of the transducers included in one of the plurality of sub-arrays. The processor 120 may control an analog beamformer and a digital beamformer to generate transmission signals respectively applied to the plurality of transducers according to the time delay values for analog beamforming and the time delay values for digital beamforming. In addition, the processor 120 may control the analog beamformer to combine the signals received from the plurality of transducers on the basis of the sub-array in accordance with the time delay values for analog beamforming. In addition, the processor 120 may control the ultrasound transceiver module 110 to perform analog-to-digital conversion on the combined signals on the basis of the sub-array. In addition, the processor 120 may control the digital beamformer to generate ultrasound data by combining the digital-converted signals in accordance with the time delay values for digital beamforming.

The image processor 130 may generate or process an ultrasound image by using the generated ultrasound data.

The display 140 may display the generated ultrasound image and a variety of information processed by the ultrasound imaging apparatus 40 or the probe 20. The probe 20 or the ultrasound imaging apparatus 40 may include one display 140 or a plurality of displays 140 depending on the type of implementation. In addition, the display 140 may include a touch panel or a touch screen. In addition, the display 140 may include a flexible display.

Examples of a mode for the ultrasound image may be amplitude mode (A-mode), brightness mode (B-mode), color Doppler mode, Doppler mode (D-mode), elastography mode (E-mode), motion mode (M-mode), and volume mode.

The processor 120 may control the overall operation of the ultrasound imaging apparatus 40 and operations of elements of the ultrasound imaging apparatus 40. The processor 120 may perform or control various operations or functions of the ultrasound imaging apparatus 40 by executing programs or instructions stored in the memory 150. In addition, the processor 120 may control the operation of the ultrasound imaging apparatus 40 upon receiving a control signal from the input interface 170 or an external device.

The ultrasound imaging apparatus 40 may include a communication module 160 and may be connected to and communicate with external devices (e.g., probe 20, ablation device 200, server, medical apparatus, and portable device (e.g., smart phone, tablet PC, and wearable device) via the communication module 160.

The communication module 160 may include at least one element enabling communication with the external devices. The communication module 160 may include, for example, at least one of a short-range communication module, a wired communication module, or a wireless communication module.

The communication module 160 may receive a control signal or data from the external devices. The processor 120 may control the operation of the ultrasound imaging apparatus 40 in accordance with the control signal received via the communication module 160. In addition, the processor 120 may transmit a control signal to an external device via the communication module 160 and control the external device in accordance with the transmitted control signal. The external device may operate according to the control signal received from the ultrasound imaging apparatus 40 or may process data received from the ultrasound imaging apparatus 40.

Programs or applications associated with the ultrasound imaging apparatus 40 may be installed in the external device. The programs or applications installed in the external device may control the ultrasound imaging apparatus 40 or may operate in accordance with the control signal or data received from the ultrasound imaging apparatus 40.

The external device may receive or download programs or applications related to the ultrasound imaging apparatus 40 from the ultrasound imaging apparatus 40, the probe 20, or a server and may install and execute the programs or applications in the external device. The ultrasound imaging apparatus 40, the probe 20, or the server providing the programs or applications may include a recording medium that stores instructions, commands, installation files, execute files, or related data of the programs or applications. The external device may also be marketed in a state with programs or applications installed therein.

The memory 150 may store various data or programs for driving and controlling the ultrasound imaging apparatus 40, input/output ultrasound data, ultrasound images, and the like.

In an embodiment, the memory 150 may store various data or programs for providing improved user experience during an ablation procedure.

The input interface 170 may receive a user input to control the ultrasound imaging apparatus 40. For example, the user input may include an input for manipulating a button, a keypad, a dial, a mouse, a trackball, a jog switch, or a knop, an input for touching a touchpad or a touch screen, a speech input, a motion input, a bio-data input (e.g., iris recognition or fingerprint recognition), and the like, but is not limited thereto.

FIG. 1B is a control block diagram of an ultrasound imaging system 100 in the case where the probe 20 is a wireless probe or a hybrid probe.

According to various embodiments of the disclosure, it should be appreciated that the ultrasound imaging apparatus 40 shown in FIG. 1B may be replaced with the ultrasound imaging apparatus 40 described above with reference to FIG. 1A.

According to various embodiments of the disclosure, it should be understood that the probe 20 shown in FIG. 1A may be replaced with the probe 20 to be described with reference to FIG. 1B.

The probe 20 may include a display 112, a transmitter module 113, a battery 114, a transducer 115, a charging module 116, a receiver module 117, an input interface 109, a processor 118, and a communication module 119. Although the probe 20 shown in FIG. 1B includes the transmitter module 113 and the receiver module 117, the probe 20 may include some elements of the transmitter module 113 and the receiver module 117, and the some elements of the transmitter module 113 and the receiver module 117 may also be included in the ultrasound imaging apparatus 40. In addition, the probe 20 according to an embodiment of the disclosure may further include an image processor 130.

The transducer 115 may include a plurality of transducers. The plurality of transducers may be arranged in a predetermined arrangement to be implemented as a transducer array. The transducer array may correspond to a one-dimensional (1D) array or a two-dimensional (2D) array. The plurality of transducers may transmit ultrasound signals to an object 10 in accordance with a transmission signal received from a transmitter module 113. Also, the plurality of transducers may generate or crease an electrical reception signal by receiving ultrasound signals (echo signals) reflected by the object 10.

The charging module 116 may charge the battery 114. The charging module 116 may receive power from the outside. According to an embodiment of the disclosure, the charging module 116 may receive powder wirelessly. In addition, according to an embodiment of the disclosure, the charging module 116 may receive power by wire. The charging module 116 may transfer the received power to the battery 114.

The processor 118 controls the transmitter module 113 to generate or create transmission signals respectively applied to each of the plurality of transducers in consideration of positions and focal points of the plurality of transducers.

The processor 118 controls the receiver module 117 to generate ultrasound data by performing analog-to-digital conversion on reception signals received from the transducer 115, and combining digital-converted reception signals in consideration of positions and focal points of the plurality of transducers. According to an embodiment of the disclosure, in the case where the probe 20 includes the image processor 130, an ultrasound image may be generated by using the generated ultrasound data.

In the case where the probe 20 is implemented as a 2D probe, the processor 118 may calculate a time delay value for digital beamforming of each of the plurality of sub-arrays included in the 2D transducer array on the basis of the sub-array. In addition, the processor 118 may calculate a time delay value for analog beamforming of each of the transducers included in one of the plurality of sub-arrays. The processor 118 may control an analog beamformer and a digital beamformer to generate transmission signals respectively applied to the plurality of transducers according to the time delay values for analog beamforming and the time delay values for digital beamforming. In addition, the processor 118 may control the analog beamformer to combine the signals received from the plurality of transducers on the basis of the sub-array in accordance with the time delay values for analog beamforming. In addition, the processor 118 may control the ultrasound transceiver module 110 to perform analog-to-digital conversion on the combined signals on the basis of the sub-array. In addition, the processor 118 may control the digital beamformer to generate ultrasound data by combining the digital-converted signals in accordance with the time delay values for digital beamforming.

The processor 118 may control the overall operation of the probe 20 and operations of elements of the probe 20. The processor 118 may perform or control various operations or functions of the probe 20 by executing programs or instructions stored in the memory 111. In addition, the processor 118 may control the operation of the probe 20 upon receiving a control signal from the input interface 109 of the probe 20 or an external device (e.g.: ultrasound imaging apparatus 40). The input interface 109 may receive a user input to control the probe 20. For example, the user input may include an input for manipulating a button, a keypad, a mouse, a trackball, a jog switch, or a knop, an input for touching a touchpad or a touch screen, a speech input, a motion input, a bio-data input (e.g., iris recognition or fingerprint recognition), and the like, but is not limited thereto.

The display 112 may display an ultrasound image generated by the probe 20, an ultrasound image generated by processing the ultrasound data generated by the probe 20, an ultrasound image received from the ultrasound imaging apparatus 40, or a variety of information processed in the ultrasound imaging system 100. In addition, the display 112 may further display status information of the probe 20. The status information of the probe 20 may include at least one of device information of the probe 20, battery status information of the probe 20, frequency band information of the probe 20, output information of the probe 20, abnormality information of the probe 20, setting information of the probe 20, and temperature information of the probe 20.

The probe 20 may include one display 112 or a plurality of displays 112 depending on the type of implementation. In addition, the display 112 may include a touch panel or a touch screen. In addition, the display 112 may include a flexible display.

The communication module 119 may transmit the generated ultrasound data or an ultrasound image to the ultrasound imaging apparatus 40 via a wireless network. In addition, the communication module 119 may receive the control signal and data from the ultrasound imaging apparatus 40.

The ultrasound imaging apparatus 40 may receive ultrasound data or an ultrasound image from the probe 20.

In an embodiment of the disclosure, in the case where the probe 20 includes the image processor 130 capable of generating an ultrasound image by using ultrasound data, the probe 20 may transmit ultrasound data of an ultrasound image generated by the image processor 130 to the ultrasound imaging apparatus 40.

In an embodiment of the disclosure, in the case where the probe 20 does not include the image processor 130 capable of generating an ultrasound image by using ultrasound data, the probe 20 may transmit ultrasound data to the ultrasound imaging apparatus 40. The ultrasound data may include ultrasound raw data, and the ultrasound image may refer to ultrasound image data.

The ultrasound imaging apparatus 40 may include a processor 120, an image processor 130, a display 140, a memory 150, a communication module 160, and an input interface 170.

The image processor 130 generates or processes an ultrasound image by using ultrasound data received from the probe 20.

The display 140 may display an ultrasound image received from the probe 20, an ultrasound image generated by processing ultrasound data received from the probe 20, a variety of information processed in the ultrasound imaging system 100 or the like. The ultrasound imaging apparatus 40 may include one display 140 or a plurality of displays 140 depending on the type of implementation. In addition, the display 140 may include a touch panel or a touch screen. In addition, the display 140 may include a flexible display.

The processor 120 may control the overall operation of the ultrasound imaging apparatus 40 and operations of elements of the ultrasound imaging apparatus 40. The processor 120 may perform or control various operations or functions of the ultrasound imaging apparatus 40 by executing programs or applications stored in the memory 150. In addition, the processor 120 may control the operation of the ultrasound imaging apparatus 40 upon receiving a control signal from the input interface 170 or an external device.

The ultrasound imaging apparatus 40 may include a communication module 160 and may be connected to and communicate with external devices (e.g., probe 20, server, medical apparatus, or portable device (e.g., smart phone, tablet PC, and wearable device) via the communication module 160.

The communication module 160 may include at least one element enabling communication with an external device. The communication module 160 may include, for example, at least one of a short-range communication module, a wired communication module, or a wireless communication module.

The communication module 160 of the ultrasound imaging apparatus 40 may communicate with the communication module 119 of the probe 20 via a network or a short-range wireless communication method. For example, the communication module 160 of the ultrasound imaging apparatus 40 may communicate with the communication module 119 of the probe 20 via one of the wireless communication methods: wireless LAN, Wi-Fi, Bluetooth, ZigBee, Wi-Fi Direct (WFD), infrared data association (IrDA), Bluetooth Low Energy (BLE), Near Field Communication (NFC), Wireless Broadband Internet (WiBro), World Interoperability for Microwave Access (WiMAX), Shared Wireless Access Protocol (SWAP), Wireless Gigabit Alliance (WiGig), radio frequency (RF) communication, or 60 GHz mm Wave short-range communication.

To this end, the communication module 160 of the ultrasound imaging apparatus 40 and the communication module 119 of the probe 20 may include at least one of a wireless LAN communication module, a Wi-Fi communication module, a Bluetooth communication module, a Zigbee communication module, a Wi-Fi Direct (WFD) communication module, an infrared data association (IrDA) module, a Bluetooth LowEnergy (BLE) communication module, a near field communication (NFC) module, a Wireless Broadband Internet (Wibro) communication module, a World Interoperability for Microwave Access (WiMAX) communication module, a Shared Wireless Access Protocol (SWAP) communication module, a Wireless Gigabit Allicance (WiGig) communication module, a RF communication module, or a 60 GHz mm Wave short-range communication module.

In an embodiment of the disclosure, the probe 20 may transmit device information (e.g., ID information) of the probe 2 to the ultrasound imaging apparatus 40 by using a first communication method (e.g.: BLE) and may be paired wirelessly with the ultrasound imaging apparatus 40. The probe 20 may also transmit ultrasound data and/or an ultrasound image to the paired ultrasound imaging apparatus 40.

The device information of the probe 20 may include various information related to serial number, model name, or battery status of the probe 20.

The ultrasound imaging apparatus 40 may receive the device information (e.g., ID information) of the probe 2 from the probe 20 by using the first communication method (e.g.: BLE) and may be paired with the probe 20 wirelessly. Also, the ultrasound imaging apparatus 40 may transmit an activation signal to the paired probe 20 and receive the ultrasound data and/or ultrasound image from the probe 20. In this regard, the activation signal may include a signal to control the operation of the probe 20.

In an embodiment of the disclosure, the probe 20 may transmit device information (e.g., ID information) of the probe 2 to the ultrasound imaging apparatus 40 by using a first communication method (e.g.: BLE) and may be paired wirelessly with the ultrasound imaging apparatus 40. Also, the probe 20 may transmit the ultrasound data and/or ultrasound image to the ultrasound imaging apparatus 40 paired by the first communication method by using a second communication method (e.g.: 60 GHz mm Wave and Wi-Fi).

The ultrasound imaging apparatus 40 may receive the device information (e.g., ID information) of the probe 2 from the probe 20 by using the first communication method (e.g.: BLE) and may be paired with the probe 20 wirelessly. Also, the ultrasound imaging apparatus 40 may transmit an activation signal to the paired probe 20 and receive the ultrasound data and/or ultrasound image from the probe 20 by using the second communication method (e.g.: 60 GHz mm Wave and Wi-Fi).

According to an embodiment of the disclosure, the first communication method used for pairing of the probe 20 and the ultrasound imaging apparatus 40 may have a frequency band lower than that of the second communication method used by the probe 20 to transmit the ultrasound data and/or ultrasound image to the ultrasound imaging apparatus 40.

The display 140 of the ultrasound imaging apparatus 40 may display a user interface (UI) indicating device information of the probe 20. For example, the display 140 may display a user interface (UI) indicating identification information of the wireless ultrasound probe 20, a pairing method for pairing with the probe 20, a data communication status between the probe 20 and the ultrasound imaging apparatus 40, a data communication method with the ultrasound imaging apparatus 40, or a battery status of the probe 20.

In the case where the probe 20 includes the display 112, the display 112 of the probe 20 may display an UI indicating device information of the probe 20. For example, the display 112 may display a user interface (UI) indicating identification information of the wireless ultrasound probe 20, a pairing method for pairing with the probe 20, a data communication status between the probe 20 and the ultrasound imaging apparatus 40, a data communication method with the ultrasound imaging apparatus 40, or a battery status of the probe 20.

The communication module 160 may receive a control signal or data from an external device. The processor 120 may control the operation of the ultrasound imaging apparatus 40 in accordance with the control signal received via the communication module 160.

In addition, the processor 120 may transmit a control signal to the external device via the communication module 160 to control the external device in accordance with the transmitted control signal. The external device may operate in accordance with the control signal received from the ultrasound imaging apparatus 40 or process data received from the ultrasound imaging apparatus 40.

The external device may receive or download programs or applications related to the ultrasound imaging apparatus 40 from the ultrasound imaging apparatus 40, the probe 20, or a server and may install and execute the programs or applications in the external device. The ultrasound imaging apparatus 40, the probe 20, or the server providing the programs or applications may include a recording medium that stores instructions, commands, installation files, execute files, or related data of the programs or applications. The external device may also be marketed in a state with programs or applications installed therein.

The memory 150 may store various data or programs for driving and controlling the ultrasound imaging apparatus 40, input/output ultrasound data, ultrasound images, and the like.

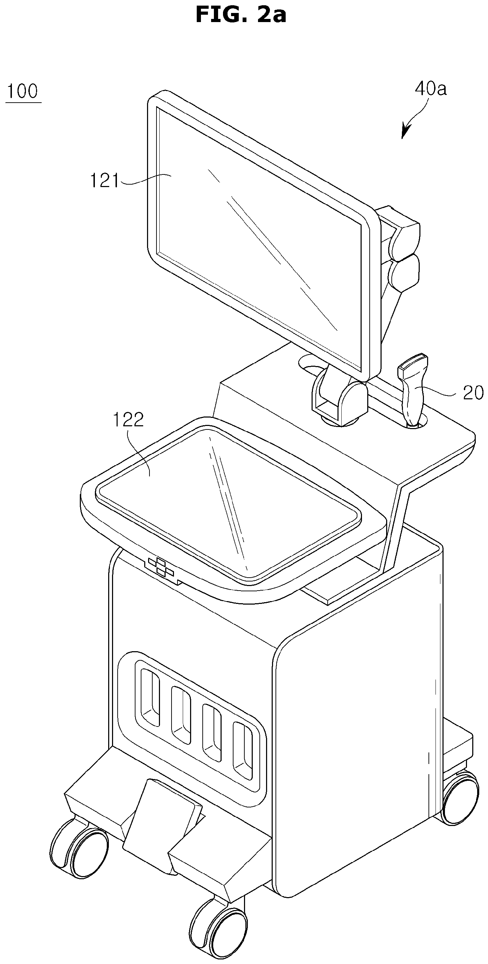

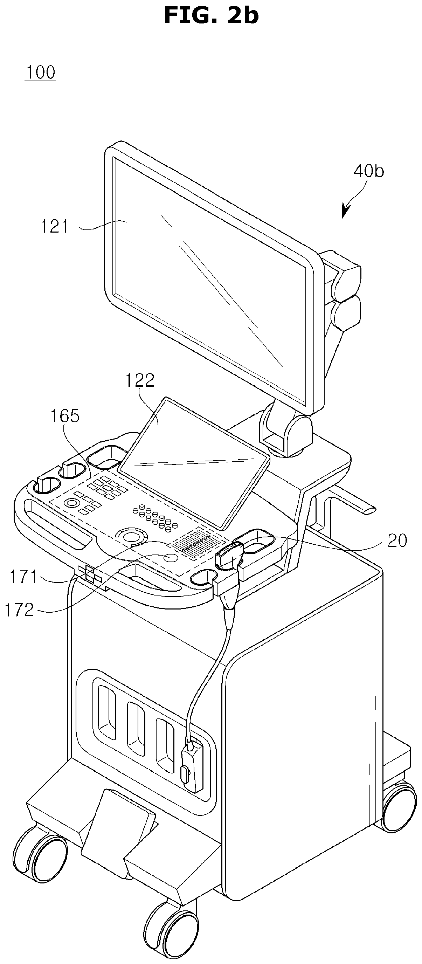

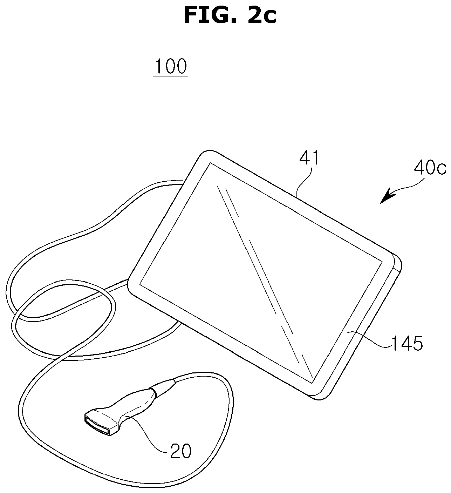

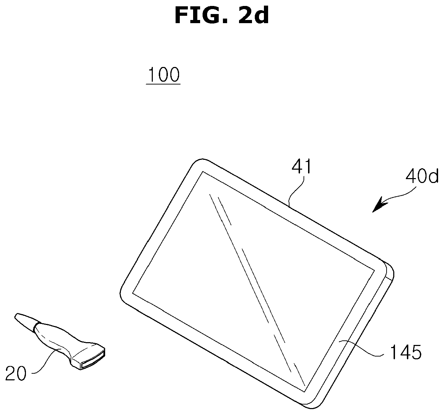

Examples of the ultrasound imaging system 100 according to an embodiment of the disclosure will be described below with reference to FIGS. 2A, 2B, 2C, and 2D.

FIGS. 2A, 2B, 2C, and 2D are diagrams illustrating ultrasound imaging apparatuses according to an embodiment of the disclosure.

Referring to FIGS. 2A and 2B, ultrasound imaging apparatuses 40a and 40b may include a main display 121 and a sub-display 122. The main display 121 and the sub-display 122 may correspond to the display 140 of FIGS. 1A and 1B. At least one of the main display 121 or the sub-display 122 may be implemented as a touch screen. At least one of the main display 121 or the sub-display 122 may display an ultrasound image or various information processed by the ultrasound imaging apparatuses 40a and 40b. In addition, at least one of the main display 121 or the sub-display 122 is implemented as a touch screen and provides a graphic user interface (GUI) to receive data for controlling the ultrasound imaging apparatuses 40a and 40b from a user. For example, the main display 121 may display an ultrasound image, and the sub-display 122 may display a control panel to control the display of the ultrasound image in the form of a GUI. The sub-display 122 may receive data for controlling the displaying of an image via a control panel displayed in the form of a GUI. For example, a time gain compensation (TGC) button, a lateral gain compensation (LGC) button, a freeze button, a trackball, a jog switch, or a knop may be provided on the sub-display 122 as a GUI.

The ultrasound imaging apparatuses 40a and 40b may control the displaying of the ultrasound image on the main display 121 by using the received input control data. In addition, the ultrasound imaging apparatuses 40a and 40b may be connected to the probe 20 by wire or wirelessly to transmit/receive an ultrasound signal to/from an object.

Referring to FIG. 2B, the ultrasound imaging apparatus 40b may further include a control panel 165 in addition to the main display 121 and the sub-display 122. The control panel 165 may include a button, a trackball, a jog switch, a knop, or the like and may receive an input of data for controlling the ultrasound imaging apparatus 40b. For example, the control panel 165 may include a TGC button 171, a freeze button 172, or the like. The TGC button 171 is a button for setting a TGC value according to a depth of an ultrasound image. Upon detection of an input of the freeze button 172 while scanning the ultrasound image, the ultrasound imaging apparatus 40b may maintain a state of displaying of a frame image of a corresponding time point, capture the frame image of the time point, or store the frame image of the time point.

Meanwhile, the button, the trackball, the jog switch, the knop, or the like included in the control panel 165 may be provided on the main display 121 or the sub-display 122 as a GUI. In addition, the ultrasound imaging apparatuses 40a and 40b may be connected to the probe 20 and may transmit/receive an ultrasound signal to/from an object.

In addition, the ultrasound imaging apparatuses 40a and 40b may include various types of input/output interfaces such as a speaker, an LED, and a vibration device. For example, the ultrasound imaging apparatuses 40a and 40b may output various information in the form of graphic, sound, or vibration via the input/output interface. In addition, the ultrasound imaging apparatuses 40a and 40b may output various notifications or data via the input/output interface.

Referring to FIGS. 2C and 2D, the ultrasound imaging apparatuses 40c and 40d may be implemented as portable devices. Examples of the portable ultrasound imaging apparatuses 40c and 40d may be a smart phone, a laptop computer, a PDA, or a tablet PC, each including a probe and applications, but are not limited thereto.

The ultrasound imaging apparatus 40c may include a main body 41. Referring to FIG. 2C, the probe 20 may be connected to one side of the main body 41 by wire. To this end, the main body 41 may include a connector to which a cable connected to the probe 20 is detachably coupled. The probe 20 may include a cable including a connector coupled to the main body 41.

Referring to FIG. 2D, the probe 20 may be connected to the ultrasound imaging apparatus 40d wirelessly. The main body 41 may include an input/output interface (e.g.: touch screen). The input/output interface may display an ultrasound image, various information processed in the ultrasound imaging apparatus, a GUI, or the like.

The ultrasound imaging apparatus 40d and the probe 20 may communicate or be paired with each other via short-range wireless communication. For example, the ultrasound imaging apparatus 40d and the probe 20 may communicate with each other via Bluetooth, BLE, Wi-Fi, Wi-Fi Direct, or the like.

The ultrasound imaging apparatuses 40c and 40d may control the probe 20 and output information related to the probe 20 by executing a program or application related to the probe 20. The ultrasound imaging apparatuses 40c and 40d may perform an operation related to the probe 20 while communicating with a certain server. The probe 20 may be registered in the ultrasound imaging apparatuses 40c and 40d or in a server. The ultrasound imaging apparatuses 40c and 40d may communicate with the registered probe 20 and perform an operation related to the probe 20.

In addition, the ultrasound imaging apparatuses 40c and 40d may include various types of input/output interfaces such as a speaker, an LED, and a vibration device. For example, the ultrasound imaging apparatuses 40c and 40d may output various information in the form of graphic, sound, or vibration via the input/output interface. In addition, the ultrasound imaging apparatuses 40c and 40d may output various notifications or data via the input/output interface.

According to an embodiment of the disclosure, the ultrasound imaging apparatus 40a, 40b, 40c, or 40d may process an ultrasound image or obtain additional information from the ultrasound image by using an artificial intelligence (Al) model. According to an embodiment of the disclosure, the ultrasound imaging apparatus 40a, 40b, 40c, or 40d may generate an ultrasound image or process the ultrasound image by correction, image quality improvement, encoding, or decoding using the AI model. In addition, according to an embodiment of the disclosure, the ultrasound imaging apparatus 40a, 40b, 40c, or 40d may process the ultrasound image, for example, may perform baseline definition, anatomical information acquisition, lesion information acquisition, surface extraction, boundary definition, length measurement, area measurement, volume measurement, or annotation creation by using the AI model.

The AI model may be included in the ultrasound imaging apparatus 40a, 40b, 40c, or 40d, or a server.

The AI model may be implemented by using various artificial neural network or deep neural network models. Also, the AI model may be trained or generated by various machine learning algorithms or deep learning algorithms. The AI model may be implemented, for example, by using convolutional neural network (CNN), recurrent neural network (RNN), generative adversarial network (GAN), long short-term memory (LSTM), or the like.

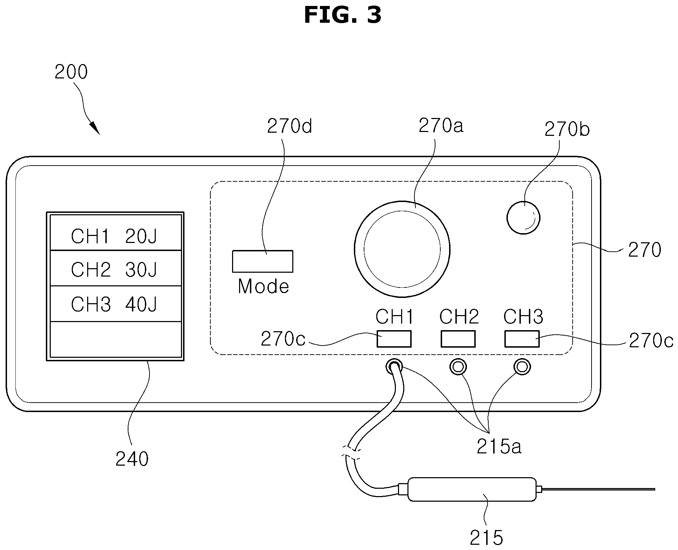

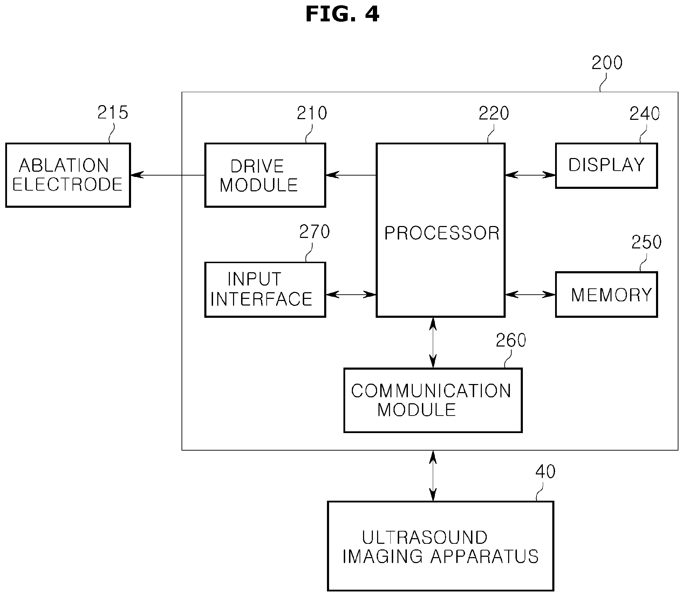

FIG. 3 is a diagram illustrating an ablation device according to an embodiment of the disclosure. FIG. 4 is a block diagram illustrating a configuration of the ablation device according to an embodiment of the disclosure.

Referring to FIGS. 3 and 4, an ablation device 200 according to an embodiment may be electrically connected to an ablation electrode 215 and apply an electrical energy (voltage or current) to the ablation electrode 215.

Because the ablation device 200 generates radiofrequency currents (or radiofrequency voltages) applied to the ablation electrode 215, the ablation device 200 may also be referred to as a radiofrequency generator.

According to various embodiments, the ablation device 200 may be included in one element of the ultrasound imaging apparatus 40.

The ablation device 200 may include at least one port 215a for connection with the ablation electrode 215.

Various types of the ablation electrodes 215 may be connected to the ablation device 200 via the at least one port 215a. The ablation device 200 may apply an electrical energy (radiofrequency voltage or radiofrequency current) to the ablation electrode 215 connected to the at least one port 215a.

Applying the electrical energy to the ablation electrode 215 may include applying a radiofrequency current to the ablation electrode 215.

Applying the electrical energy to the ablation electrode 215 may include a radiofrequency voltage to the ablation electrode 215.

The at least one port 215a may also be referred to as at least one of the channels 215a (CH1, CH2, and CH3).

The ablation device 200 may include a user interface device (e.g.: display 240 and/or input interface 270).

The display 240 may display various information related to the ablation device 200.

For example, the display 240 may display status information of the ablation electrode 215 connected to each of the channels 215a.

The status information of the ablation electrode 215 may include information on set output of the ablation electrode 215, information on temperature of the ablation electrode 215, information on impedance of the ablation electrode 215, and information on a total output value output by the ablation electrode 215.

The set output of the ablation electrode 215 may be a magnitude of an electrical energy set to be applied via the ablation electrode 215. In the disclosure, for descriptive convenience, the set output of the ablation electrode 215 will be described using joules (J) as a unit of the electrical energy.

A user may identify the status information of the ablation electrode 215 via the display 240.

The input interface 270 may receive an input of a user to control the ablation device 200. For example, the input of the user may include an input for manipulating a button, a keypad, a dial, a mouse, a trackball, a jog switch, or a knop, an input for touching a touchpad or a touch screen, a speech input, a motion input, and a bio-data input (e.g., iris recognition or fingerprint recognition), but is not limited thereto.

The input interface 270 may include a first input device 270a configured to allow a user to select a channel to control among the at least one channel and receive a user input for setting an output of the selected channel. The first input device 270a may be implemented in the form of a dial, but the form of the first input device 270a is not limited thereto.

The user may select a channel to control via the input interface 270 and set an output of the selected channel.

The input interface 270 may include a second input device 270b configured to turn on/off the power of the ablation device 200. The second input device 270b may be implemented in the form of a button, but the form of the second input device 270b is not limited thereto.

The input interface 270 may include a third input device 270c configured to activate at least one of the channels CH1, CH2, and CH3. The third input device 270c may be implemented in the form of a button, but the form of the third input device 270c is not limited thereto.

The activating of the at least one of the channels CH1, CH2, and CH3 may include applying an electrical energy to the ablation electrode 215 connected to the at least one of the channels CH1, CH2, and CH3.

That is, after setting the output of each of the channels CH1, CH2, and CH3 via the input interface 270, the user may input a command for applying an electrical energy (e.g.: voltage) to the ablation electrode 215 as a set output.

A command for applying the electrical energy (e.g.: voltage) to the ablation electrode 215 as the set output may also be referred to as a current inject command.

Only upon receiving the current inject command, the ablation device 200 may apply the electrical energy to the ablation electrode 215 as the set output.

In the disclosure, the 'set output of the ablation electrode 215' may refer to an output of a channel set by the user.

For example, in the case where the user sets an output of a first channel CH1 to '20J', the set output of the ablation electrode 215 connected to the first channel CH1 may be '20J'.

The user may ablate a target point by setting an output of the ablation electrode 215, inserting the ablation electrode 215 into the target point of the object 10, and inputting a command for applying a voltage to the ablation electrode 215.

The input interface 270 may include a fourth input device 270d configured to change a mode of the ablation device 200. The fourth input device 270dc may be implemented in the form of a button, but the form of the fourth input device 270d is not limited thereto.

The mode of the ablation device 200 may include, for example, a single switching mode and a bipolar mode.

The single switching mode is a mode in which each of the ablation electrodes 215 connected to the at least one of the channels CH 1, CH2, and CH3 operates as a positive electrode. In the case where the ablation device 200 operates in the single switching mode, a negative pad should be attached to a body part (e.g., thigh) of the object 10. The negative pad may also be connected to one of the channels CH1, CH2, and CH3.

The negative pad may also be called as a ground pad because the negative pad provides a reference potential (or ground potential) for the ablation electrode 215.

Once a radiofrequency voltage is applied to the ablation electrode 215, a radiofrequency current may be applied to the ablation electrode 215 due to a potential difference between the ground pad and the ablation electrode 215.

The bipolar mode is a mode in which one ablation electrode 215 connected to one of the channels CH1, CH2, and CH3 operates as a positive electrode, and another ablation electrode 215 connected to another channel of the channels CH1, CH2, and CH3 operates as a negative electrode. In the case where the ablation device 200 operates in the bipolar mode, the negative pad is not required to be attached to the body part of the object 10.

In the case where the ablation device 200 operates in the bipolar mode, the user may insert both the ablation electrode 215 operating as the positive electrode (hereinafter, referred to as 'positive electrode') and the ablation electrode 215 operating as the negative electrode (hereinafter, referred to as 'negative electrode' or 'ground electrode') into the object 10, and ablate a lesion by using a potential difference formed between the positive electrode and the negative electrode.

That is, once a radiofrequency voltage is applied to the positive electrode, a radiofrequency current may be applied to the ablation electrode 215 due to a potential difference between the negative electrode and the positive electrode.

In the case where the display 240 is implemented as a touch screen, the input interface 270 may include a touch screen.

The processor 220 may control the overall operation of the ablation device 200 and control operations of elements of the ablation device 200. The processor 220 may perform or control various operations or functions of the ablation device 200 by executing programs or instructions stored in the memory 250. In addition, the processor 220 may control the operation of the ablation device 200 upon receiving a control signal from the input interface 170 or an external device.

The ablation device 200 may include a communication module 260 and may be connected to and communicate with external devices (e.g., ultrasound imaging apparatus 40) via the communication module 260.

The communication module 260 may include at least one element enabling communication with an external device. The communication module 260 may include, for example, at least one of a short-range communication module, a wired communication module, or a wireless communication module.

The ablation device 200 may transmit status information of the ablation electrode 215 to the ultrasound imaging apparatus 40 via the communication module 260.

The status information of the ablation electrode 215 may include information on a set output of an ablation electrode 215 connected to the at least one of the channels CH1, CH2, and CH3.

That is, the ultrasound imaging apparatus 40 may receive information on the set output of the ablation electrode 215 from the ablation device 200.

In the disclosure, the connecting of the ultrasound imaging apparatus 40 with the ablation electrode 215 may include that connecting the ultrasound imaging apparatus 40 with the ablation device 200 by wire or wirelessly.

In the disclosure, the connecting of the ultrasound imaging apparatus 40 with the ablation electrode 215 may include that the ultrasound imaging apparatus 40 may receive status information of the ablation electrode 215 from the ablation device 200.

The drive module 210 may drive the ablation electrode 215. Driving of the ablation electrode 215 may include applying an electrical energy to the ablation electrode 215.

The drive module 210 may apply an electrical energy corresponding to the set output of the ablation electrode 215 connected to at least one of the channels CH1, CH2, and CH3 under the control of the processor 220.

To this ends, the drive module 210 may include a radiofrequency generating circuit capable of generating radiofrequency power.

The ablation electrode 215 may ablate a lesion upon receiving the electrical energy received from the ablation device 200. The ablation electrode 215 may also be referred to as an ablation probe, ablation needle, and the like.

The ablation electrode 215 may include a handle grabbable by a user and a needle insertable into the object 10.

The needle insertable into the object 10, as a positive electrode, may receive a radiofrequency voltage.

The needle insertable into the object 10, as a ground electrode, may provide a ground potential for the positive electrode.

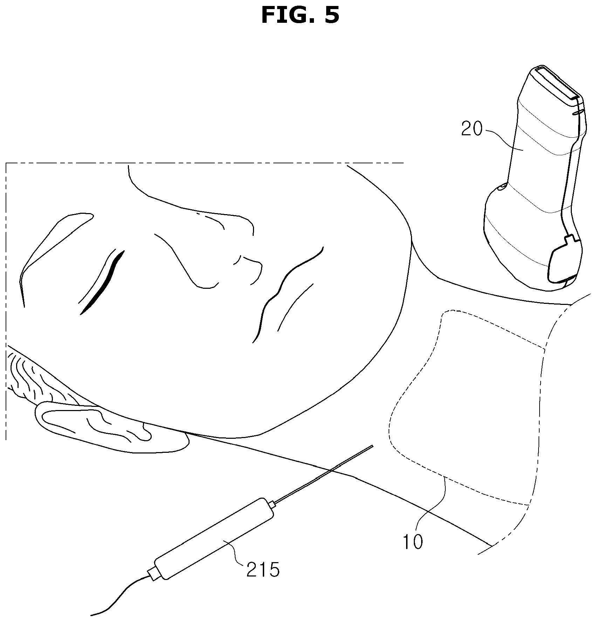

FIG. 5 is a diagram illustrating that an ablation procedure is performed on an object by using an ablation electrode and an ultrasound probe.

The ablation procedure may be performed mainly to remove a lesion in the thyroid gland or liver, but the purpose of the ablation procedure is not limited thereto.

The user may perform an ablation procedure by using the ultrasound probe 20 and the ablation electrode 215.

More specifically, the user may locate the ablation electrode 215 at the lesion while observing an ultrasound image Im obtained via the ultrasound probe 20, and ablate an area around the ablation electrode 215 by applying an electrical energy to the ablation electrode 215.

The locating of the ablation electrode 215 on the lesion may include locating an end of the ablation electrode 215 on the lesion. Once a current is applied to the ablation electrode 215, the current spread into the body via the end of the ablation electrode 215, and thus an area around the end of the ablation electrode 215 is ablated.

Meanwhile, the user may proceed with the procedure while judging an area expected to be ablated by the ablation electrode 215 (hereinafter, referred to as 'ablation expected area') based on intuition or experience of the user.

Inexperienced users have great difficulties in performing the ablation procedure because it is difficult to intuitively judge the ablation expected area.

An embodiment of the disclosure provides the ultrasound imaging apparatus 40 providing improved user experience while proceeding with the ablation procedure and a method for controlling the ultrasound imaging apparatus 40, and thus inexperienced users may receive great help in performing the ablation procedure.

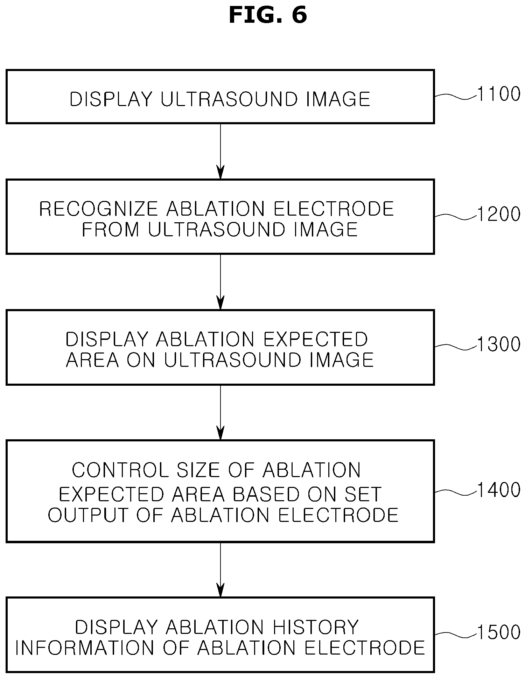

FIG. 6 is a flowchart illustrating an example of a method for controlling an ultrasound imaging apparatus according to an embodiment of the disclosure.

Referring to FIG. 6, a method for controlling the ultrasound imaging apparatus 40 may include an operation of displaying an ultrasound image Im performed by the ultrasound imaging apparatus 40 (1100).

The processors 120 and 130 may display the ultrasound image Im generated based on echo signals received by the ultrasound probe 20 on the display 140.

For example, the processors 120 and 130 may generate an ultrasound image Im based on echo signals received by the ultrasound probe 20 and display the generated ultrasound image Im on the display 140.

As another example, the processor 118 of the ultrasound probe 20 may generate an ultrasound image Im based on echo signals received by the ultrasound probe 20 and transmit the ultrasound image Im to the ultrasound imaging apparatus 40, and the processors 120 and 130 may display the ultrasound image Im received from the ultrasound probe 20 on the display 140.

According to various embodiments, in the case of performing an ablation procedure, the user may change a mode of the ultrasound imaging apparatus 40 to an ablation mode via the input interface 170.

According to various embodiments, in the ablation mode, the ultrasound imaging apparatus 40 may perform operations described below.

However, according to various embodiments, even when the ablation mode is not selected, the ultrasound imaging apparatus 40 may perform the operations described below upon recognition of the ablation electrode 215 in the ultrasound image Im or upon connection of the ultrasound imaging apparatus 40 to the ablation electrode 215.

The method for controlling the ultrasound imaging apparatus 40 may include an operation of recognizing the ablation electrode 215 from the ultrasound image Im (1200).

The recognition of the ablation electrode 215 from the ultrasound image Im may include detecting the ablation electrode 215 from the ultrasound image Im.

The processors 120 and 130 may recognize the ablation electrode 215 from the ultrasound image Im.

For example, the processors 120 and 130 may perform image pre-processing on the ultrasound image Im.

The image pre-processing may include one or more processes of converting the ultrasound image Im into a form more suitable for recognizing the ablation electrode 215. For example, the image pre-processing may include a process of removing noise from the ultrasound image Im, a process of increasing a contrast of the ultrasound image Im, a deblurring process of eliminating blur from the ultrasound image Im, a process of removing a background region, a warping process of correcting distortion included in the ultrasound image Im, a process of binarizing the ultrasound image Im, and the like.

The processors 120 and 130 detect the ablation electrode 215 in the ultrasound image Im. The processors 120 and 130 may detect the ablation electrode 215 in the ultrasound image Im by using, for example, a Haar-based cascade adaboost classifier, a neural network-based classifier, or a support vector machine. However, the embodiments are not limited thereto, and the processors 120 and 130 may detect the ablation electrode 215 in the ultrasound image Im by using various methods for detecting the ablation electrode 215.

In the disclosure, the 'ablation electrode 215' may refer to an actual ablation electrode 215, and the 'recognized ablation electrode' (RAE) may refer to an ablation electrode detected in the ultrasound image Im. In the disclosure, the 'recognized ablation electrode RAE' may refer to an image of the ablation electrode 215 in the ultrasound image Im.

The processors 120 and 130 may receive status information of the ablation electrode 215 from the ablation device 200.

The status information of the ablation electrode 215 may include various information such as information on set output of the ablation electrode 215, information on temperature of the ablation electrode 215, information on impedance of the ablation electrode 215, and information on a total output value output by the ablation electrode 215.

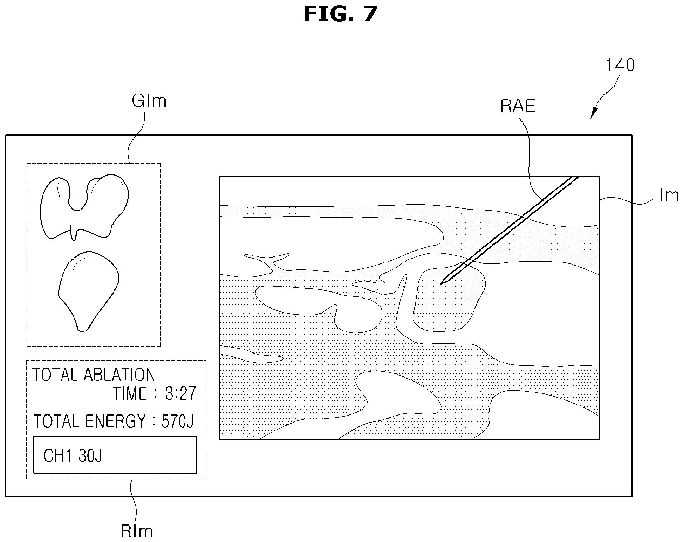

FIG. 7 illustrates an example of an interface provided by an ultrasound imaging apparatus according to an embodiment of the disclosure.

Referring to FIG. 7, the processors 120 and 130 may display status information Rlm of the ablation electrode 215 on the display 140.

As shown in the drawing, the status information Rlm of the ablation electrode 215 may include information on a set output of the ablation electrode 215, information on a total energy applied to the ablation electrode 215, a total ablation time during which the ablation is performed by using the ablation electrode 215, and the like, but the status information Rlm of the ablation electrode 215 is not limited thereto.

According to various embodiments, the processors 120 and 130 may display a guide image Glm that visualizes the object to be ablated. For example, in the case where the mode of the ultrasound imaging apparatus 40 is changed to the ablation mode, the user may input a command for selecting the object to be ablated via the input interface 170.

That is, the ultrasound imaging apparatus 40 may display the guide image Glm visualizing the object to be ablated selected by the user input.

The processors 120 and 130 may display the guide image Glm visualizing the object to be ablated together with the ultrasound image Im.

The guide image Glm visualizing the object to be ablated may previously be stored in the memory 150 to correspond to various objects to be ablated.

According to various embodiments, the processors 120 and 130 may identify the lesion in the object to be ablated based on the processing of the ultrasound image Im and modify the guide image Glm such that the lesion region is visually distinguished from the other regions.

According to various embodiments, the processors 120 and 130 may identify the lesion in the object to be ablated based on the processing of the ultrasound image Im and modify the ultrasound image Im such that the lesion region is visually distinguished from the other regions.

The guide image Glm visualizing the object to be ablated may include an image of the object to be ablated itself or an image of a lesion identified in the object to be ablated.

According to various embodiments, the processors 120 and 130 may apply various visual effects to the recognized ablation electrode RAE in the ultrasound image Im so that the recognized ablation electrode RAE is distinguished from the other regions of the object 10.

For example, the processors 120 and 130 may change the color of the recognized ablation electrode RAE to be different from that of the other regions or may indicate the border of the recognized ablation electrode RAE with a thick line or a dashed line to distinguish the border from the other regions.

The method for controlling the ultrasound imaging apparatus 40 may include an operation of displaying an ablation expected area REA of the recognized ablation electrode RAE on the ultrasound image Im performed by the ultrasound imaging apparatus 40 (1300).

The displaying of the ablation expected area REA on the ultrasound image Im may include indicating the ablation expected area REA on the ultrasound image Im.

The display of the ablation expected area REA on the ultrasound image Im may include overlapping a visual indicator indicating the ablation expected area REA on the ultrasound image Im.

The ablation expected area REA of the recognized ablation electrode RAE may refer to an area expected to be ablated in the case of applying an electrical energy corresponding to the set output to the ablation electrode 215.

The operation of displaying the ablation expected area REA of the recognized ablation electrode RAE on the ultrasound image Im performed by the ultrasound imaging apparatus 40 (1300) may include an operation of determining the ablation expected area REA based on a position of the recognized ablation electrode RAE. In the disclosure, the position of the ablation electrode 215 may include a position of an end of the ablation electrode 215. In this regard, the end of the ablation electrode 215 may refer to a tip of the needle of the ablation electrode 215.

The processors 120 and 130 may determine the ablation expected area REA based on the position of the recognized ablation electrode RAE.

The processors 120 and 130 may display a visual indicator corresponding to the ablation expected area REA.

The processors 120 and 130 may control the size of the visual indicator based on the set output of the ablation electrode 215.

The processors 120 and 130 may display the ablation expected area REA based on the position of the recognized ablation electrode RAE. For example, in the case where the ultrasound image Im is a 3D image, the processors 120 and 130 may determine a spherical area centered at the end of the recognized ablation electrode RAE with a preset radius as the ablation expected area REA. In the disclosure, the sphere may include a spheroid.

In the case where the sphere centered at the end of the recognized ablation electrode RAE is determined as the ablation expected area REA, the processors 120 and 130 may display the ablation expected area REA with a spherical visual indicator.

In an embodiment, in the case where the ultrasound image Im is a 2D image, the processors 120 and 130 may determine a circular region centered at the end of the recognized ablation electrode RAE with a preset radius as the ablation expected area REA. In the disclosure, the circle may include an ellipse.

In the case where the circle centered at the end of the recognized ablation electrode RAE is determined as the ablation expected area REA, the processors 120 and 130 may display the ablation expected area REA with a circular visual indicator.

The method for controlling the ultrasound imaging apparatus 40 may include an operation of controlling a size of the ablation expected area REA performed by the ultrasound imaging apparatus 40 (1400).

The operation of controlling a size of the ablation expected area REA (1400) may be included in the operation of displaying the ablation expected area REA (1300).

The controlling of the size of the ablation expected area REA may include determining, adjusting, changing, or modifying the size of the ablation expected area REA.

The controlling of the size of the ablation expected area REA may include determining, adjusting, changing, or modifying the shape of the ablation expected area REA.

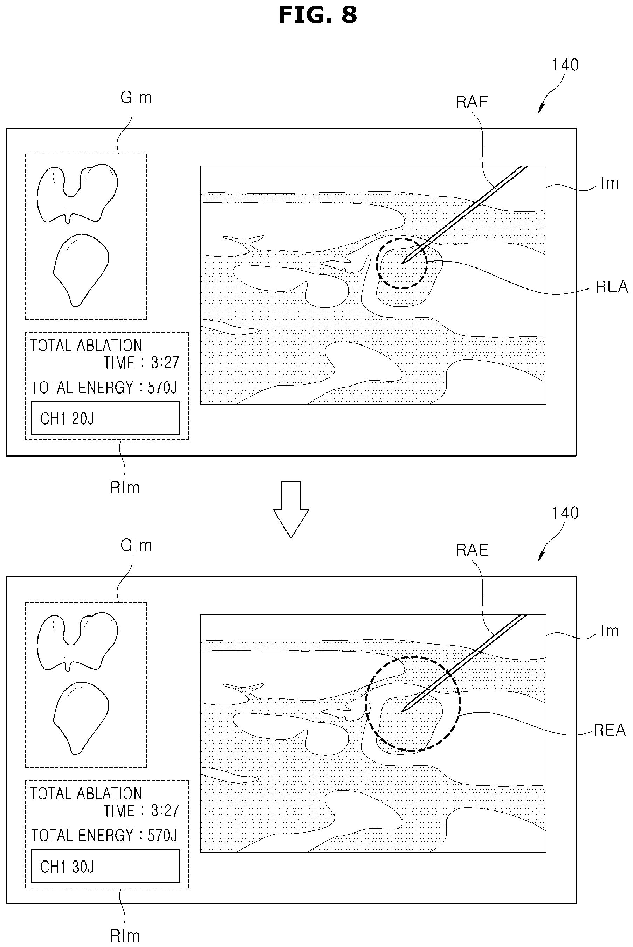

FIG. 8 is a diagram illustrating a change in the size of an ablation expected area displayed by the ultrasound imaging apparatus according to an embodiment of the disclosure.

The processors 120 and 130 may control the size of the ablation expected area REA based on the set output of the ablation electrode 215.

The processors 120 and 130 may change the size of the ablation expected area REA in response to a change in the set output of the ablation electrode 215.

For example, the processors 120 and 130 may increase the size of the ablation expected area REA as the set output of the ablation electrode 215 increases.

In an embodiment, the processors 120 and 130 may linearly increase or decrease the size of the ablation expected area REA according to the set output of the ablation electrode 215.

In the case where the set output of the ablation electrode 215 is less than a preset value, the size of the ablation expected area REA tends to linearly increase as an intensity of the set output increases. Accordingly, in the case where the set output of the ablation electrode 215 is less than a preset value, the processors 120 and 130 may linearly increase or decrease the size of the ablation expected area REA according to the set output of the ablation electrode 215. However, according to various embodiments, regardless of whether the set output of the ablation electrode 215 exceeds a preset value or not, the processors 120 and 130 may linearly increase or decrease the size of the ablation expected area REA according to the set output of the ablation electrode 215.

In an embodiment, the processors 120 and 130 may non-linearly increase or decrease the size of the ablation expected area REA according to the set output of the ablation electrode 215.

In the case where the set output of the ablation electrode 215 is a preset value or more, the size of the ablation expected area REA tends to non-linearly increase as the intensity of the set output increases. For example, because the ablation expected area REA has a spherical shape, a volume of the ablation expected area REA corresponds to a cube of a radius, a distance from the end of the ablation electrode 215 to an external angle of the spherical shape, and accordingly, the set output may increase not linearly but non-linearly to ablate an area with a larger volume. Accordingly, in the case where the set output of the ablation electrode 215 is greater than a preset value, the processors 120 and 130 may non-linearly increase or decrease the size of the ablation expected area REA according to the set output of the ablation electrode 215. However, according to various embodiments, regardless of whether the set output of the ablation electrode 215 exceeds the preset value or not, the processors 120 and 130 may non-linearly increase or decrease the size of the ablation expected area REA according to the set output of the ablation electrode 215.

Referring to FIG. 8, in the case where the set output of the ablation electrode 215 is changed from 20 J to 30 J, it may be confirmed that the size of the ablation expected area REA increases.

Increasing of a size of the ablation expected area REA as the set output of the ablation electrode 215 increases may include decreasing the size of the ablation expected area REA as the set output of the ablation electrode 215 decreases.

In the case where the ablation expected area REA is displayed with a spherical visual indicator, the processors 120 and 130 may control the radius of the sphere based on the set output of the ablation electrode 215.

Meanwhile, the processors 120 and 130 may determine the ablation expected area REA as a spheroid as the set output of the ablation electrode 215 increases. For example, as the set output of the ablation electrode 215 increases, a length of a portion where electric charges are concentrated in the ablation electrode 215 increases, and the center of the sphere may move from one end of the ablation electrode 215 in a direction toward the other end of the ablation electrode 215. In this regard, the direction toward the other end of the ablation electrode 215 may be a direction toward the handle of the ablation electrode 215.

In the case where the center of the sphere moves from one end of the ablation electrode 215 to the other end of the ablation electrode 215, the sphere may become a spheroid with a major axis in a lengthwise direction of the ablation electrode 215.

That is, the processors 120 and 130 may change the shape of the ablation expected area REA based on the set output of the ablation electrode 215.

Determining of the radius of the sphere based on the set output of the ablation electrode 215 may include determining a radius in the major axis and/or a radius in the minor axis of the sphere based on the set output of the ablation electrode 215.

The increasing of the size of the ablation expected area REA as the set output of the ablation electrode 215 increases may include increasing the radius of the sphere based on the set output of the ablation electrode 215.

The increasing of the radius of the sphere based on the set output of the ablation electrode 215 may include increasing the radius in the major axis and/or the radius in the minor axis of the sphere based on the set output of the ablation electrode 215.

In the case where the ablation expected area REA is displayed as a circular visual indicator, the processors 120 and 130 may control the radius of the circle based on the set output of the ablation electrode 215.

Meanwhile, the processors 120 and 130 may determine the ablation expected area REA as an ellipse as the set output of the ablation electrode 215 increases. For example, as the set output of the ablation electrode 215 increases, a length of a portion of the ablation electrode 215 where electric charges are concentrated increases, and the center of the circle may move from one end of the ablation electrode 215 to the other end of the ablation electrode 215.

In the case where the center of the circle moves from one end of the ablation electrode 215 to the other end of the ablation electrode 215, the circle may become an ellipse with a major axis in a lengthwise direction of the ablation electrode 215.

That is, the processors 120 and 130 may change the shape of the ablation expected area REA based on the set output of the ablation electrode 215.

Determining the radius of the circle based on the set output of the ablation electrode 215 may include determining a radius in the major and/or a radius in the minor axis of the ellipse based on the set output of the ablation electrode 215.

The increasing of the size of the ablation expected area REA as the set output of the ablation electrode 215 increases may include increasing the radius of the circle based on the set output of the ablation electrode 215.

The increasing of the radius of the circle based on the set output of the ablation electrode 215 may include increasing the radius in the major axis and/or the radius in the minor axis of the ellipse based on the set output of the ablation electrode 215.

According to the disclosure, because the size of the ablation expected area REA is changed in accordance with the set output of the ablation electrode 215 and displayed on the display 140, the user may easily identify an area expected to be ablated according to application of the electrical energy to the ablation electrode 215.

The memory 150 may store a look-up table regarding shapes and/or sizes of the ablation expected areas REAs corresponding to the set outputs of the ablation electrode 215.

According to various embodiments, the shapes and/or sizes of the ablation expected areas REAs corresponding to the set outputs of the ablation electrode 215 may be set by the user.

For example, the user may set the shape of the ablation expected area REA corresponding to the set output of the ablation electrode 215. The memory 150 may store information on the shape of the ablation expected area REA and corresponding to the set output of the ablation electrode 215 set by the user.

As another example, the user may set the size of the ablation expected area REA corresponding to the set output of the ablation electrode 215. The memory 150 may store information on the size of the ablation expected area REA and corresponding to the set output of the ablation electrode 215 set by the user.

For example, the user may set a first size of the ablation expected area REA displayed on the display 140 in the case where the set output of the ablation electrode 215 is a first set output and may set a second size of the ablation expected area REA displayed on the display 140 in the case where the set output of the ablation electrode 215 is a second set output.

The processors 120 and 130 may control the size of the ablation expected area REA to the first size set by the user in the case where the set output of the ablation electrode 215 is the first set output, and may control the size of the ablation expected area REA to the second size set by the user in the case where the set output of the ablation electrode 215 is the second set output. Meanwhile, in the case where the first set output is lower than the second set output, the processors 120 and 130 may control the display 140 to display an error message upon determination that the first size is set to be greater than the second size by the user. In addition, the processors 120 and 130 may identify the second size corresponding to the second set output by analyzing the relationship between the first set output and the first size and provide the user with the identified second size as a recommended set size.

In an embodiment, the processors 120 and 130 may determine a size of the ablation expected area REA corresponding to a set output, which is not set by the user, based on the size of the ablation expected area REA corresponding to the set output set by the user.

For example, in the case where the first size is set to the first set output and the second size is set to the second set output by the user, the processors 120 and 130 may determine sizes of the ablation expected areas REAs of the other set outputs, which are not set by the user, by interpolating into or extrapolating from a non-linear model based on the relationships between the first set output and the first size and between the second set output and the second size.

The setting of the size of the ablation expected area REA corresponding to the set output of the ablation electrode 215 by the user may be performed by an external device or the input interface 170.

The method for controlling the ultrasound imaging apparatus 40 may include an operation of displaying ablation history information of the ablation electrode 215 performed by the ultrasound imaging apparatus 40 (1500).

The processors 120 and 130 may receive operation information of the ablation electrode 215 and display ablation history information of the ablation electrode 215 on the display 140 based on the operation information.
ablation history information may include information on an area where ablation is completed by the ablation electrode 215.

The displaying of the ablation history information of the ablation electrode 215 may include displaying the ablation history information to overlap the ultrasound image Im and/or the guide image Glm.

The processors 120 and 130 may display an area where ablation is completed by the ablation electrode 215 (hereinafter, referred to as 'ablation completed area RCA') on the ultrasound image Im and/or the guide image Glm based on the operation of the ablation electrode 215.

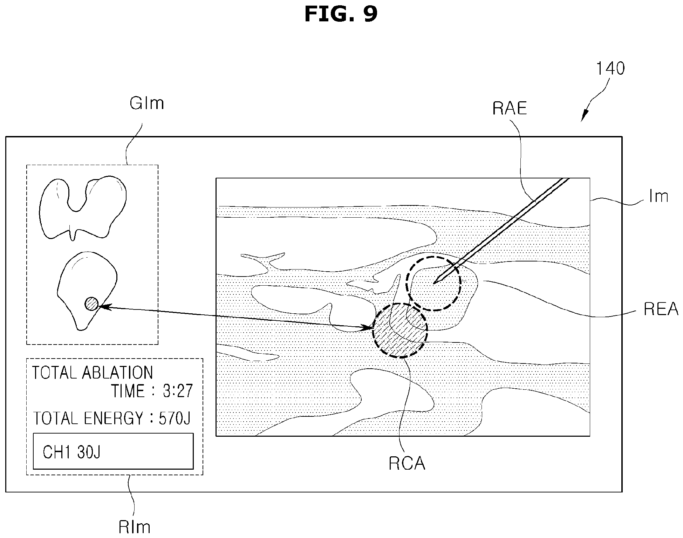

FIG. 9 is a diagram illustrating an area where ablation is completed in an ultrasound image and/or in a guide image displayed by an ultrasound imaging apparatus according to an embodiment of the disclosure.

Referring to FIG. 9, in response to application of an electrical energy corresponding to a set output to the ablation electrode 215 while displaying an ablation expected area REA, the processors 120 and 130 may determine the ablation expected area REA as an ablation completed area RCA.

A user input for applying the electrical energy to the ablation electrode 215 may be received by the input interface 270 of the ablation device 200, and the ablation device 200 may transmit information that the electrical energy was applied to the ablation electrode 215 to the ultrasound imaging apparatus 40.

In response to reception of a user input for applying the electrical energy corresponding to the set output to the ablation electrode 215 based on the set output of the ablation electrode 215 while an ablation expected area REA is displayed, the processors 120 and 130 may determine the ablation expected area REA as an ablation completed area RCA.

The processors 120 and 130 may display the ablation completed area RCA to be visually distinguished from the other areas.

That is, upon application of the electrical energy corresponding to the set output to the ablation electrode 215, the processors 120 and 130 may display the ablation expected area REA to be visually distinguished from the other areas in the ultrasound image Im.

For example, the processors 120 and 130 may provide a visually effect (shading or color) to the ablation completed area RCA in the ultrasound image Im.

According to various embodiments, the processors 120 and 130 may indicate an area of the object to be ablated corresponding to the ablation completed area RCA in the guide image Glm.

According to an embodiment of the disclosure, the user may easily identify the ablation completed area RCA, and thus a mistake of applying the electrical energy multiple times to the ablation completed area RCA may be prevented.

The displaying the ablation history information of the ablation electrode 215 may include displaying information on the ablation completed area RCA.

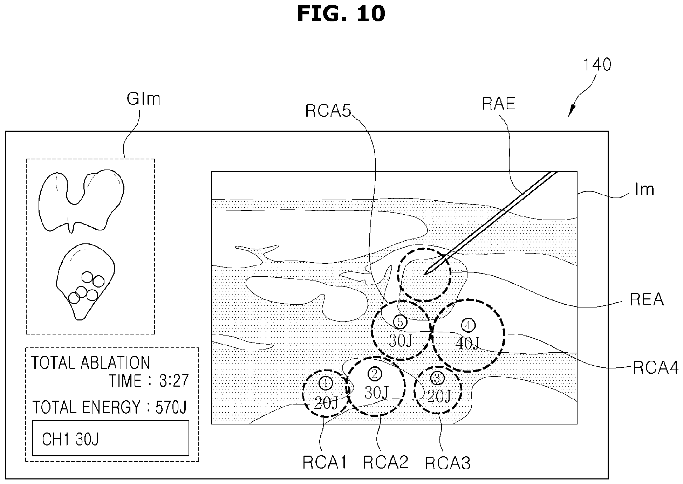

FIG. 10 is a diagram illustrating an example of ablation history information displayed by the ultrasound imaging apparatus 40 according to an embodiment of the disclosure.

Upon completion of the plurality of ablation operations performed on the object 10, a plurality of ablation completed areas RCAs may be displayed on the ultrasound image Im. In this regard, the ablation operation may refer to an operation of applying an electrical energy to the ablation electrode 215.

Referring to FIG. 10, the processors 120 and 130 may display a plurality of ablation completed areas RCA1, RCA2, RCA3, RCA4, and RCA5 corresponding to a plurality of ablation operations on the ultrasound image Im.

Furthermore, the processors 120 and 130 may display information on the plurality of ablation completed areas RCA1, RCA2, RCA3, RCA4, and RCA5 on the ultrasound image Im.

The information on the plurality of ablation completed areas RCA1, RCA2, RCA3, RCA4, and RCA5 may include information on an ablation order of the plurality of ablation completed areas RCA1, RCA2, RCA3, RCA4, and RCA5 and/or information on the electrical energy applied to the plurality of ablation completed areas RCA1, RCA2, RCA3, RCA4, and RCA5.

In an embodiment, the processors 120 and 130 may receive information on the ablation order of the ablation expected areas REAs upon application of the electrical energy corresponding to the set output to the ablation electrode 215.

Referring to FIG. 10, the processors 120 and 130 may display, on the display 140, a visual indicator (e.g., No. '1') indicating the ablation order of the first ablation completed area RCA1, ablation of which was performed first, among the plurality of ablation completed areas RCAs.

The processors 120 and 130 may display, on the display 140, a visual indicator (e.g., No. '2') indicating the ablation order of the second ablation completed area RCA2, ablation of which was performed second, among the plurality of ablation completed areas RCAs.

The processors 120 and 130 may display, on the display 140, a visual indicator (e.g., No. '3') indicating the ablation order of the third ablation completed area RCA3, ablation of which was performed third, among the plurality of ablation completed areas RCAs.

The processors 120 and 130 may display, on the display 140, a visual indicator (e.g., No. '4') indicating the ablation order of the fourth ablation completed area RCA4, ablation of which was performed fourth, among the plurality of ablation completed areas RCAs.

The processors 120 and 130 may display, on the display 140, a visual indicator (e.g., No. '5') indicating the ablation order of the fifth ablation completed area RCA5, ablation of which was performed fifth, among the plurality of ablation completed areas RCAs.

In an embodiment, the visual indicator indicating the ablation order of the ablation completed area RCA may be displayed in the ablation completed area RCA or in the guide image Glm.

Once the ablation operation is performed on the ablation expected area REA, the processors 120 and 130 represents the ablation expected area REA as an ablation completed area RCA and may display, on the display 140, a visual indicator (e.g., No. '6') indicating the ablation order of the ablation completed area RCA, ablation of which was performed sixth.

According to an embodiment of the disclosure, the user may proceed with the ablation while easily recognizing the ablation order.

Meanwhile, the function of displaying the ablation order may be freely turned on/off according to a user input.

In an embodiment, upon application of an electrical energy corresponding to a set output of the ablation electrode 215, the processors 120 and 130 may display information on the electrical energy applied to the ablation expected area REA.

The processors 120 and 130 may display, on the display 140, a visual indicator (e.g.: text '20J') indicating an electrical energy applied to the first ablation completed area RCA1 in which an ablation operation was completed with a set output of 20 J among the plurality of ablation completed areas RCAs.

The processors 120 and 130 may display, on the display 140, a visual indicator (e.g.: text '30J') indicating an electrical energy applied to the second ablation completed area RCA2 in which an ablation operation was completed with a set output of 30 J among the plurality of ablation completed areas RCAs.

The processors 120 and 130 may display, on the display 140, a visual indicator (e.g.: text '20J') indicating an electrical energy applied to the third ablation completed area RCA3 in which an ablation operation was completed with a set output of 20 J among the plurality of ablation completed areas RCAs.

The processors 120 and 130 may display, on the display 140, a visual indicator (e.g.: text '40J') indicating an electrical energy applied to the fourth ablation completed area RCA4 in which an ablation operation was completed with a set output of 40 J among the plurality of ablation completed areas RCAs.

The processors 120 and 130 may display, on the display 140, a visual indicator (e.g.: text '30J') indicating an electrical energy applied to the fifth ablation completed area RCA5 in which an ablation operation was completed with a set output of 30 J among the plurality of ablation completed areas RCAs.

In an embodiment, the visual indicators indicating the electrical energies applied to the ablation completed areas RCAs may be displayed in the ablation completed areas RCAs or on the guide image Glm.

Once the ablation operation is performed on the ablation expected area REA, the processors 120 and 130 may represent the ablation expected area REA as an ablation completed area RCA and display a visual indicator (text '30J') indicating an electrical energy applied to the ablation completed area RCA in which the ablation was completed with a set output of 30 J.

According to an embodiment of the disclosure, the user may proceed with the ablation while easily recognizing the electrical energies applied to the plurality of ablation completed areas RCAs.

Meanwhile, the function of displaying the electrical energy applied to the ablation completed area RCA may be freely turned on/off according to a user input.

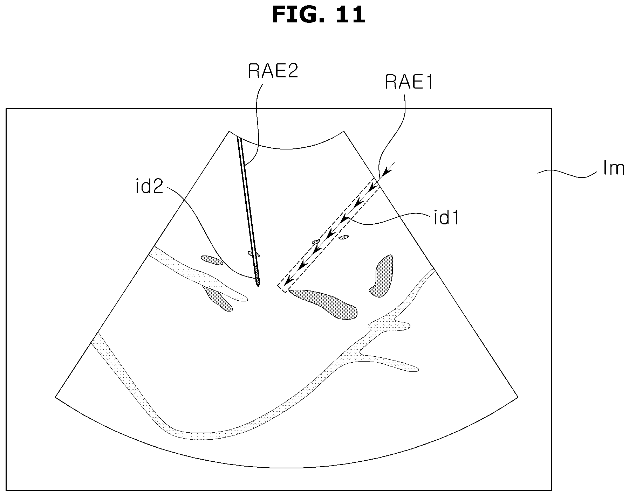

FIG. 11 is a diagram illustrating a current flow of an ablation electrode displayed by an ultrasound imaging apparatus according to an embodiment of the disclosure.

In response to reception of the current inject command, the ablation device 200 may transmit a message indicating that the current inject command has been received to the ultrasound imaging apparatus 40.

In general, although the current inject command is input by an assistant of the user, it takes some time for a current arrives at the ablation electrode 215 after the current inject command is input. In the case where the user is able to intuitively recognize a current flow in the ablation electrode 215 by observing only the ultrasound image Im, the user may recognize a time point when the current inject command is input.

In addition, in the case where the user is able to intuitively recognize the current flow in the ablation electrode 215 by observing only the ultrasound image Im, the user may recognize the ablation electrode 215 to which the current is supplied in the case of using a plurality of ablation electrodes 215.

Referring to FIG. 11, upon application of an electrical energy corresponding to a set output of the ablation electrode 215, the processors 120 and 130 may display visual indicators id1 and id2 indicating current flows in the recognized ablation electrodes RAE1 and RAE2.

According to an embodiment of the disclosure, the user may intuitively recognize one of the plurality of recognized ablation electrodes RAE1 and RAE2 where the current flows via the visual indicators id1 and id2 indicating the current flow.

According to various embodiments, the visual indicators id1 and id2 indicating current flows may include a visual indicator id1 indicating a dynamic status of the current and a visual indicator id2 indicating a static status of the current.

The processors 120 and 130 may display the visual indicator id1 indicating a dynamic status of the current at the recognized ablation electrode RAE1 matching the positive electrode among the plurality of recognized ablation electrodes RAEs and display the visual indicator id2 indicating a statis status of the current at the recognized ablation electrode RAE2 matching the negative electrode among the plurality of recognized ablation electrodes RAEs.

According to an embodiment of the disclosure, in the case where the ablation device 200 operates in the bipolar mode, the user may distinguish the positive electrode from the negative electrode via the visual indicators id1 and id2 showing current flows.

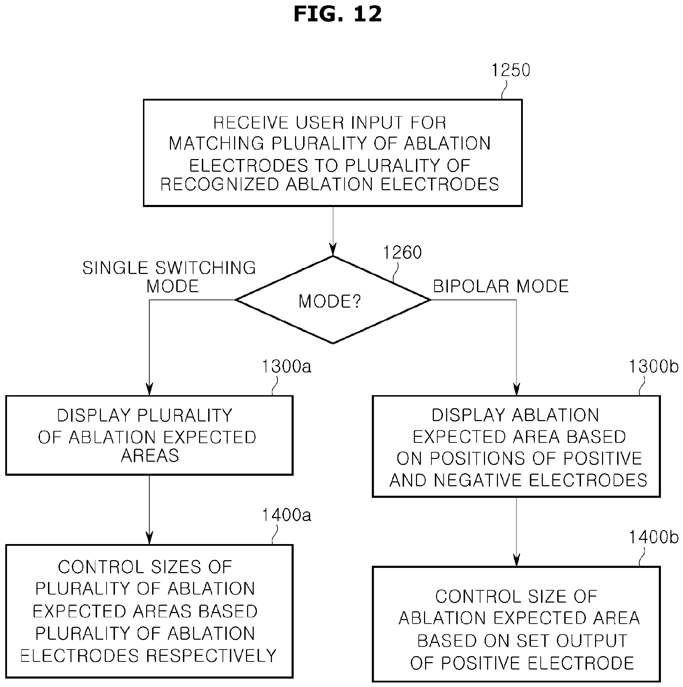

FIG. 12 is a flowchart illustrating an example of a method for controlling an ultrasound imaging apparatus according to an embodiment of the disclosure.

As described above, the user may perform the ablation procedure by using a plurality of ablation electrodes 215.

That is, the ablation electrode 215 may include a plurality of ablation electrodes 215. For example, the ablation electrode 215 may include a first ablation electrode 215 and a second ablation electrode 215.

The method for controlling the ultrasound imaging apparatus 40 may include an operation of receiving a user input for matching a plurality of recognized ablation electrodes RAEs to a plurality of ablation electrodes 215, respectively, performed by the ultrasound imaging apparatus 40 (1250).

The processors 120 and 130 may receive a user input for matching the first ablation electrode 215 and the second ablation electrode 215 to a plurality of ablation electrodes 215 recognized from the ultrasound image Im, respectively.

In the case where a plurality of ablation electrodes 215 are inserted into the object 10, the ultrasound imaging apparatus 40 may recognize the plurality of ablation electrodes 215 from the ultrasound image Im in Operation 1200 of FIG. 6, but cannot identify ports of a plurality of channels 215a connected to the recognized plurality of ablation electrodes 215.

That is, the ultrasound imaging apparatus 40 cannot identify which one of the recognized ablation electrodes 215 corresponds to the ablation electrode 215 connected to a certain channel.

In order to identify the channel connected to each of the plurality of recognized ablation electrode, the ultrasound imaging apparatus 40 may receive a user input for matching the plurality of recognized ablation electrodes 215 to the plurality of ablation electrodes 215 connected to the plurality of channels 215a.

The user input for matching the recognized plurality of ablation electrodes 215 to the plurality of ablation electrodes 215 connected to the plurality of channels 215a may be received by the ultrasound imaging apparatus 40 via various methods.

The matching of the recognized plurality of ablation electrodes 215 to the plurality of ablation electrodes 215 connected to the plurality of channels 215a may include identifying that the plurality of recognized ablation electrodes 215 correspond to the plurality of ablation electrodes 215 connected to the plurality of channels 215a, respectively.

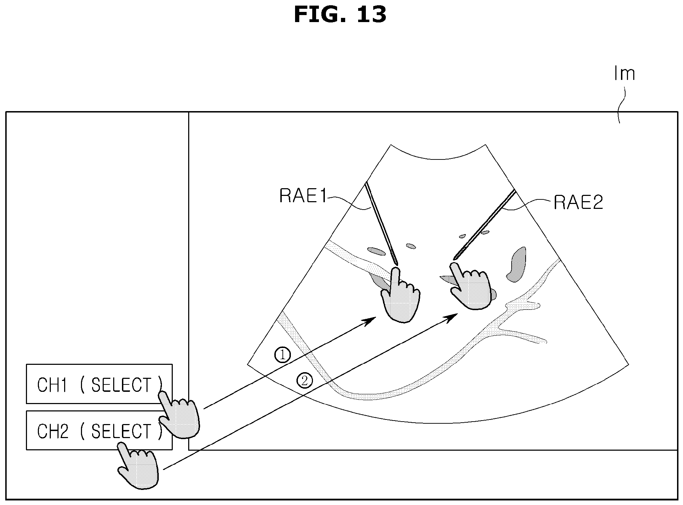

FIG. 13 illustrates an example of a method of matching a plurality of ablation electrodes to a plurality of recognized ablation electrodes by using an ultrasound imaging apparatus according to an embodiment of the disclosure.

Referring to FIG. 13, the user may match the recognized plurality of ablation electrodes 215 to the ablation electrodes 215 connected to the plurality of channels 215a by selecting one of the plurality of channels 215a connected to the ablation electrodes 215 and selecting one of the plurality of recognized ablation electrodes RAEs.

For example, the user may match the first ablation electrode 215 connected to the first channel CH1 to the first recognized ablation electrode RAE1 between the plurality of recognized ablation electrodes RAE1 and RAE2 and may match the second ablation electrode 215 connected to the second channel CH2 to the second recognized ablation electrode RAE2 between the plurality of recognized ablation electrodes RAE1 and RAE2.

The method for controlling the ultrasound imaging apparatus 40 may include an operation of identifying an operation mode of the ultrasound probe 20 performed by the ultrasound imaging apparatus 40 (1260).

The ablation device 200 may transmit information on the operation mode to the ultrasound imaging apparatus 40, and the ultrasound imaging apparatus 40 may identify the operation mode of the ablation device 200.

As described above, the operation mode of the ablation device 200 may be classified into a single switching mode and a bipolar mode.

In the case where the ablation device 200 operates in the single switching mode, both the first ablation electrode 215 and the second ablation electrode 215 may operate as positive electrodes.

In Operation 1250, the processors 120 and 130 may receive a user input for matching the first ablation electrode 215 and the second ablation electrode 215 to the plurality ablation electrodes 215 recognized from the ultrasound image Im, respectively.

Operation 1300 of FIG. 6 may include Operation 1300a of FIG. 12. That is, the operation of displaying the ablation expected area REA of the recognized ablation electrode RAE on the ultrasound image Im (1300) may include an operation of displaying the ablation expected areas REAs of the recognized plurality of ablation electrodes 215 on the ultrasound image Im (1300a).

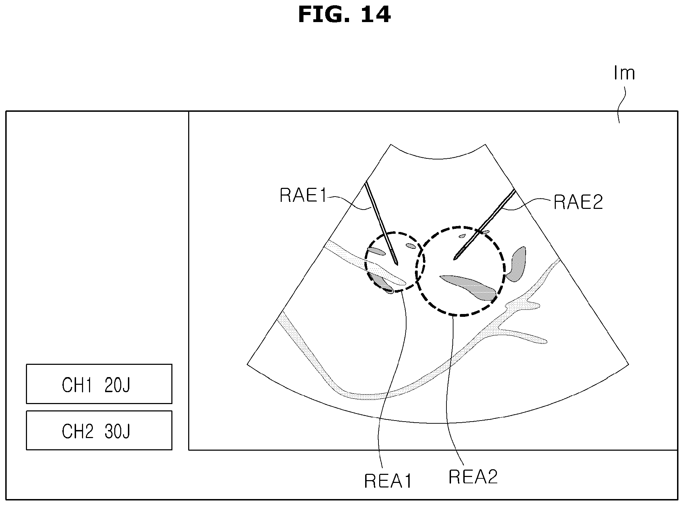

FIG. 14 illustrates a plurality of ablation expected areas with regard to a plurality of ablation electrodes displayed by an ultrasound imaging apparatus according to an embodiment of the disclosure.

Referring to FIG. 14, the processors 120 and 130 may determine a first ablation expected area REA1 based on a position of the first recognized ablation electrode RAE1 matched to the first ablation electrode 215.

The processors 120 and 130 may determine a second ablation expected area REA2 based on a position of the second recognized ablation electrode RAE2 matched to the second ablation electrode 215.

Operation 1400 of FIG. 6 may include Operation 1400a of FIG. 12. That is, the operation of controlling a size of the ablation expected area REA (1400) may include an operation of controlling sizes of the plurality of ablation expected areas REA1 and REA2 based on set outputs of the plurality of ablation electrodes 215 respectively).

The processors 120 and 130 may control the size of the first ablation expected area REA1 of the first recognized ablation electrode RAE1 matched to the first ablation electrode 215 according to the user input based on the first set output of the first ablation electrode 215.

The processors 120 and 130 may control the size of the second ablation expected area REA2 of the second recognized ablation electrode RAE2 matched to the second ablation electrode 215 according to the user input based on the second set output of the second ablation electrode 215.

According to an embodiment of the disclosure, even when the user uses a plurality of ablation electrodes 215, the plurality of ablation expected areas REA1 and REA2 expected to be ablated respectively by the plurality of ablation electrode 215 may be confirmed.

In the case where the ablation device 200 operates in the bipolar mode, the first ablation electrode 215 and the second ablation electrode 215 may operate as a positive electrode and a negative electrode, respectively.

In Operation 1250, the processors 120 and 130 may receive a user input for matching the positive electrode and the negative electrode to the plurality of ablation electrodes 215 recognized from the ultrasound image Im, respectively.

Operation 1300 of FIG. 6 may include Operation 1300b of FIG. 12. That is, the operation of displaying the ablation expected area REA of the recognized ablation electrode RAE on an ultrasound image Im (1300) may include an operation of displaying the ablation expected area REA based on the positions of the recognized positive electrode and negative electrode on the ultrasound image Im (1300b).

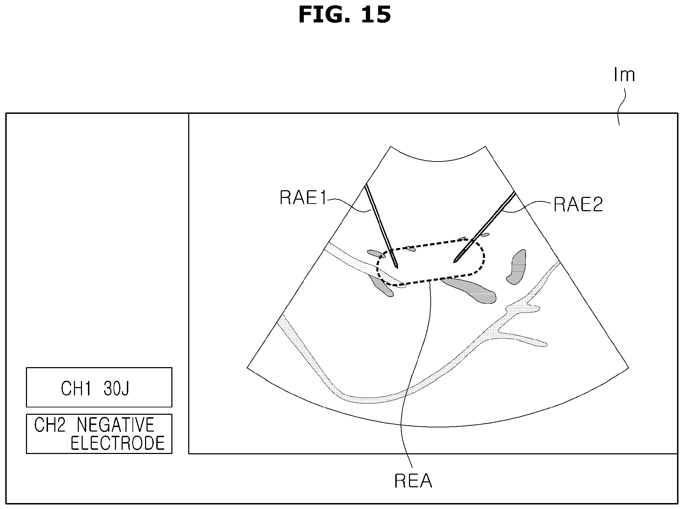

FIG. 15 is a diagram illustrating one ablation expected area with regard to a plurality of ablation electrodes displayed by an ultrasound imaging apparatus according to an embodiment of the disclosure.

Referring to FIG. 15, the processors 120 and 130 may determine an ablation expected area REA based on a position of a first recognized ablation electrode RAE1 matched to the positive electrode and a position of a second recognized ablation electrode RAE2 matched to the negative electrode. In this regard, the positions of the first and second recognized ablation electrodes RAE1 and RAE2 may include positions of ends of the first and second recognized ablation electrodes RAE1 and RAE2.

In an embodiment, the processors 120 and 130 may determine an elliptical area centered at a center of a line between ends of the first and second recognized ablation electrodes RAE1 and RAE2 as an ablation expected area REA.

Operation 1400 of FIG. 6 may include Operation 1400b of FIG. 12. That is the operation of controlling a size of the ablation expected area REA (1400) may include an operation of controlling a size of the ablation expected area REA based on the set output of the positive electrode (1400b).

The processors 120 and 130 may control the size of the ablation expected area REA based on the set output of the positive electrode.

In an embodiment, the processors 120 and 130 may control the size of the ablation expected area REA based on the set output of the positive electrode and a distance between ends of the first and second recognized ablation electrodes RAE1 and RAE2.

According to an embodiment of the disclosure, in the case where the user uses the bipolar mode, the user may identify the ablation expected area REA expected to be ablate by the positive electrode and the negative electrode.

Meanwhile, according to various embodiments, the method for controlling the ultrasound imaging apparatus 40 may be performed by external computing device.

The disclosed embodiments may be implemented in the form of a recording medium storing computer-executable instructions that are executable by a processor. The instructions may be stored in the form of a program code, and when executed by a processor, the instructions may generate a program module to perform operations of the disclosed embodiments. The recording medium may be implemented non-transitory as a computer-readable recording medium.

The computer-readable recording medium includes all kinds of recording media storing instructions that may be interpreted by a computer. Examples of computer-readable recording media include Read Only Memory (ROM), Random Access Memory (RAM), magnetic tape, magnetic disk, flash memory, and optical data storage device.

In addition, a computer-readable storage medium may be provided in the form of a non-transitory storage medium. Here, the term 'non-transitory storage medium' merely refers to a tangible device and excludes a signal (e.g., an electromagnetic wave), but the term does not distinguish between a case where data is semipermanently stored in the storage medium and a case where data is temporarily stored in the storage medium. For example, the 'non-transitory storage medium' may include a buffer in which data is temporarily stored.

According to an embodiment of the disclosure, the methods according to various embodiments disclosed herein may be included in a computer program product and provided. The computer program product may be a product and may be traded between a seller and a purchaser. The computer program product may be distributed in the form of a storage medium (e.g., a compact disc read only memory (CD-ROM)) readable by a device, or may be distributed online (e.g., downloaded or uploaded) through an application store (e.g., Play Store^{™}) or directly between two user devices (e.g., smart phones). In the case of online distribution, at least a part of a computer program product (e.g., a downloadable app) may be temporarily stored or temporarily generated in a storage medium readable by a device such as a memory of a manufacturer's server, an application store's server, or a relay server.

According to one embodiment, a practitioner may perform an ablation procedure while confirming an ablation expected area.

According to one embodiment, a practitioner may efficiently remove only a lesion.

According to one embodiment, even an inexperienced medical personnel may perform an ablation procedure.

According to one embodiment, a practitioner may efficiently remove only a lesion.

According to one embodiment, it is possible to prevent a practitioner from performing ablation in overlapping areas.

The embodiments disclosed with reference to the accompanying drawings have been described above. It will be understood by those skilled in the art that various changes in form and details may be made therein without departing from the spirit and scope of the disclosure as defined by the appended claims. The disclosed embodiments are illustrative and should not be construed as limiting.

## Claims

1. A medical imaging apparatus comprising:
a display; and
a processor configured to:
display a medical image on the display,
recognize an ablation electrode from the medical image,
display an ablation expected area of the recognized ablation electrode on the medical image, and
control a size of the ablation expected area based on a set output of the ablation electrode.

2. The medical imaging apparatus according to claim 1, wherein the processor is configured to increase the size of the ablation expected area as the set output of the ablation electrode increases.

3. The medical imaging apparatus according to claim 1, wherein the processor is configured to display a visual indicator corresponding to the ablation expected area, and
control a size of the visual indicator based on the set output of the ablation electrode.

4. The medical imaging apparatus according to claim 1, wherein based on an electrical energy corresponding to the set output being applied to the ablation electrode, the processor is configured to display the ablation expected area to be visually distinguished from the other areas in the medical image.

5. The medical imaging apparatus according to claim 1, wherein based on an electrical energy corresponding to the set output being applied to the ablation electrode, the processor is configured to display information on an ablation order of the ablation expected area.

6. The medical imaging apparatus according to claim 1, wherein based on an electrical energy corresponding to the set output being applied to the ablation electrode, the processor is configured to display information on the electrical energy applied to the ablation expected area.

7. The medical imaging apparatus according to claim 1, wherein based on an electrical energy corresponding to the set output being applied to the ablation electrode, the processor is configured to display a visual indicator indicating a current flow on the recognized ablation electrode.

8. The medical imaging apparatus according to claim 1, wherein
the ablation electrode comprises a first ablation electrode and a second ablation electrode, and
the processor is further configured to:
receive a user input for matching each of the first ablation electrode and the second ablation electrode to each of a plurality of ablation electrodes recognized from the medical image,
control a size of a first ablation expected area of a first recognized ablation electrode matched to the first ablation electrode according to the user input based on a first set output of the first ablation electrode, and
control a size of a second ablation expected area of a second recognized ablation electrode matched to the second ablation electrode according to the user input based on a second set output of the second ablation electrode.

9. The medical imaging apparatus according to claim 1, wherein
the ablation electrode comprises a positive electrode and a negative electrode, and
the processor is further configured to:
receive a user input for matching each of the positive electrode and the negative electrode to each of a plurality of ablation electrodes recognized from the medical image,
display the ablation expected area based on a position of the first recognized ablation electrode matched to the positive electrode according to the user input and a position of the second recognized ablation electrode matched to the negative electrode according to the user input, and
control a size of the ablation expected area based on the set output of the positive electrode.

10. The medical imaging apparatus according to claim 1, wherein
the processor is further configured to:
display a guide image visualizing an object to be ablated on the display together with the medical image, and
display an ablation completed area on the guide image based on an operation of the ablation electrode.

11. A method for controlling a medical imaging apparatus, the method comprising:
displaying a medical image;
recognizing an ablation electrode from the medical image;
displaying an ablation expected area of the recognized ablation electrode on the medical image; and
controlling a size of an ablation expected area based on a set output of the ablation electrode.

12. The method according to claim 11, wherein the controlling of the size of the ablation expected area comprises increasing the size of the ablation expected area as the set output of the ablation electrode increases.

13. The method according to claim 11, wherein the displaying of the ablation expected area comprises displaying a visual indicator corresponding to the ablation expected area, and
the controlling of the size of the ablation expected area comprises controlling a size of the visual indicator based on the set output to the ablation electrode.

14. The method according to claim 11, further comprising displaying the ablation expected area to be visually distinguished from the other areas in the medical image based on an electrical energy corresponding to the set output being applied to the ablation electrode.

15. The method according to claim 11, further comprising based on an electrical energy corresponding to the set output being applied to the ablation electrode, displaying at least one of information on an ablation order of the ablation expected area, information on an electrical energy applied to the ablation expected area, or a visual indicator indicating a current flow on the recognized ablation electrode.

## Amended claims

### Amended claims in accordance with Rule 137(2) EPC.

**1.** A medical imaging apparatus comprising:
a display (140); and
a processor (120) configured to:
receive a medical image,
display the medical image on the display (140),
receive information on a set output of an ablation electrode (215),
recognize the ablation electrode (215) from the medical image,
determine an ablation expected area of the recognized ablation electrode (215) based on a position of the recognized ablation electrode (215),
display the ablation expected area of the recognized ablation electrode (215) on the medical image, and
control a size of the ablation expected area displayed on the medical image based on the set output of the ablation electrode (215),
wherein based on an electrical energy corresponding to the set output being applied to the ablation electrode (215), the processor (120) is configured to display information on an ablation order of the ablation expected area.

**2.** The medical imaging apparatus according to claim 1, wherein the processor (120) is configured to increase the size of the ablation expected area as the set output of the ablation electrode (215) increases.

**3.** The medical imaging apparatus according to claim 1, wherein the processor (120) is configured to display a visual indicator corresponding to the ablation expected area, and
control a size of the visual indicator based on the set output of the ablation electrode (215).

**4.** The medical imaging apparatus according to claim 1, wherein based on the electrical energy corresponding to the set output being applied to the ablation electrode (215), the processor (120) is further configured to display the ablation expected area to be visually distinguished from the other areas in the medical image.

**5.** The medical imaging apparatus according to claim 1, wherein based on an electrical energy corresponding to the set output being applied to the ablation electrode (215), the processor (120) is configured to display information on the electrical energy applied to the ablation expected area.

**6.** The medical imaging apparatus according to claim 1, wherein based on the electrical energy corresponding to the set output being applied to the ablation electrode (215), the processor (120) is further configured to display a visual indicator indicating a current flow on the recognized ablation electrode.

**7.** The medical imaging apparatus according to claim 1, wherein
the ablation electrode (215) comprises a first ablation electrode and a second ablation electrode, and
the processor (120) is further configured to:
receive a user input for matching each of the first ablation electrode and the second ablation electrode to each of a plurality of ablation electrodes recognized from the medical image,
control a size of a first ablation expected area of a first recognized ablation electrode matched to the first ablation electrode according to the user input based on a first set output of the first ablation electrode, and
control a size of a second ablation expected area of a second recognized ablation electrode matched to the second ablation electrode according to the user input based on a second set output of the second ablation electrode.

**8.** The medical imaging apparatus according to claim 1, wherein
the ablation electrode (215) comprises a positive electrode and a negative electrode, and
the processor (120) is further configured to:
receive a user input for matching each of the positive electrode and the negative electrode to each of a plurality of ablation electrodes recognized from the medical image,
display the ablation expected area based on a position of the first recognized ablation electrode matched to the positive electrode according to the user input and a position of the second recognized ablation electrode matched to the negative electrode according to the user input, and
control a size of the ablation expected area based on the set output of the positive electrode.

**9.** A medical imaging apparatus comprising:
a display (140); and
a processor (120) configured to:
receive a medical image,
display the medical image on the display (140),
receive information on a set output of an ablation electrode (215),
recognize the ablation electrode (215) from the medical image,
determine an ablation expected area of the recognized ablation electrode (215) based on a position of the recognized ablation electrode (215),
display the ablation expected area of the recognized ablation electrode (215) on the medical image, and
control a size of the ablation expected area displayed on the medical image based on the set output of the ablation electrode (215),
wherein the processor (215) is further configured to:
display a guide image visualizing an object to be ablated on the display together with the medical image, and
display an ablation completed area on the guide image based on an operation of the ablation electrode (215).

**10.** A method for controlling a medical imaging apparatus, the method comprising:
receiving, by a processor (120), a medical image;
displaying, by the processor (120), a medical image on a display (140);
receiving, by the processor (120), information on a set output of an ablation electrode (215);
recognizing, by the processor (120), the ablation electrode (215) from the medical image;
determining, by the processor (120), an ablation expected area of the recognized ablation electrode (215) based on a position of the recognized ablation electrode (215),
displaying, by the processor (120), the ablation expected area of the recognized ablation electrode (215) on the medical image;
controlling, by the processor (120), a size of the ablation expected area displayed on the medical image based on the set output of the ablation electrode (215); and
based on an electrical energy corresponding to the set output being applied to the ablation electrode (215), displaying, by the processor (120), information on an ablation order of the ablation expected area.

**11.** The method according to claim 10, wherein the controlling of the size of the ablation expected area comprises increasing the size of the ablation expected area as the set output of the ablation electrode (215) increases.

**12.** The method according to claim 10, wherein the displaying of the ablation expected area comprises displaying a visual indicator corresponding to the ablation expected area, and
the controlling of the size of the ablation expected area comprises controlling a size of the visual indicator based on the set output to the ablation electrode (215).

**13.** The method according to claim 10, further comprising displaying the ablation expected area to be visually distinguished from the other areas in the medical image based on an electrical energy corresponding to the set output being applied to the ablation electrode (215).

**14.** The method according to claim 10, further comprising based on the electrical energy corresponding to the set output being applied to the ablation electrode (215), displaying, by the processor (120), at least one of information on an electrical energy applied to the ablation expected area, or a visual indicator indicating a current flow on the recognized ablation electrode (215).

**1.** A medical imaging apparatus comprising:
a display (140); and
a processor (120) configured to:
receive a medical image,
display the medical image on the display (140),
receive information on a set output of an ablation electrode (215),
recognize the ablation electrode (215) from the medical image,
determine an ablation expected area of the recognized ablation electrode (215) based on a position of the recognized ablation electrode (215),
display the ablation expected area of the recognized ablation electrode (215) on the medical image, and
control a size of the ablation expected area displayed on the medical image based on the set output of the ablation electrode (215),
wherein:
a) based on an electrical energy corresponding to the set output being applied to the ablation electrode (215), the processor (120) is configured to display information on an ablation order of the ablation expected area, and/or
b) the processor (120) is further configured to display a guide image visualizing an object to be ablated on the display (140) together with the medical image, and display an ablation completed area on the guide image based on an operation of the ablation electrode (215).

**2.** The medical imaging apparatus according to claim 1, wherein the processor (120) is configured to increase the size of the ablation expected area as the set output of the ablation electrode (215) increases.

**3.** The medical imaging apparatus according to claim 1, wherein the processor (120) is configured to display a visual indicator corresponding to the ablation expected area, and
control a size of the visual indicator based on the set output of the ablation electrode (215).

**4.** The medical imaging apparatus according to claim 1, wherein based on the electrical energy corresponding to the set output being applied to the ablation electrode (215), the processor (120) is further configured to display the ablation expected area to be visually distinguished from the other areas in the medical image.

**5.** The medical imaging apparatus according to claim 1, wherein based on an electrical energy corresponding to the set output being applied to the ablation electrode (215), the processor (120) is configured to display information on the electrical energy applied to the ablation expected area.

**6.** The medical imaging apparatus according to claim 1, wherein based on the electrical energy corresponding to the set output being applied to the ablation electrode (215), the processor (120) is further configured to display a visual indicator indicating a current flow on the recognized ablation electrode (215).

**7.** The medical imaging apparatus according to claim 1, wherein
the ablation electrode (215) comprises a first ablation electrode and a second ablation electrode, and
the processor (120) is further configured to:
receive a user input for matching each of the first ablation electrode and the second ablation electrode to each of a plurality of ablation electrodes recognized from the medical image,
control a size of a first ablation expected area of a first recognized ablation electrode matched to the first ablation electrode according to the user input based on a first set output of the first ablation electrode, and
control a size of a second ablation expected area of a second recognized ablation electrode matched to the second ablation electrode according to the user input based on a second set output of the second ablation electrode.

**8.** The medical imaging apparatus according to claim 1, wherein
the ablation electrode (215) comprises a positive electrode and a negative electrode, and
the processor (120) is further configured to:
receive a user input for matching each of the positive electrode and the negative electrode to each of a plurality of ablation electrodes recognized from the medical image,
display the ablation expected area based on a position of the first recognized ablation electrode matched to the positive electrode according to the user input and a position of the second recognized ablation electrode matched to the negative electrode according to the user input, and
control a size of the ablation expected area based on the set output of the positive electrode.

**9.** A method for controlling a medical imaging apparatus, the method comprising:
receiving, by a processor (120), a medical image;
displaying, by the processor (120), a medical image on a display (140);
receiving, by the processor (120), information on a set output of an ablation electrode (215);
recognizing, by the processor (120), the ablation electrode (215) from the medical image;
determining, by the processor (120), an ablation expected area of the recognized ablation electrode (215) based on a position of the recognized ablation electrode (215),
displaying, by the processor (120), the ablation expected area of the recognized ablation electrode (215) on the medical image; and
controlling, by the processor (120), a size of the ablation expected area displayed on the medical image based on the set output of the ablation electrode (215),
wherein the method further includes:
a) based on an electrical energy corresponding to the set output being applied to the ablation electrode (215), displaying information on an ablation order of the ablation expected area, and/or
b) displaying a guide image visualizing an object to be ablated on the display together with the medical image, and displaying an ablation completed area on the guide image based on an operation of the ablation electrode (215).

**10.** The method according to claim 9, wherein the controlling of the size of the ablation expected area comprises increasing the size of the ablation expected area as the set output of the ablation electrode (215) increases.

**11.** The method according to claim 9, wherein the displaying of the ablation expected area comprises displaying a visual indicator corresponding to the ablation expected area, and
the controlling of the size of the ablation expected area comprises controlling a size of the visual indicator based on the set output to the ablation electrode (215).

**12.** The method according to claim 9, further comprising displaying the ablation expected area to be visually distinguished from the other areas in the medical image based on an electrical energy corresponding to the set output being applied to the ablation electrode (215).

**13.** The method according to claim 9, further comprising based on the electrical energy corresponding to the set output being applied to the ablation electrode (215), displaying, by the processor, at least one of information on an electrical energy applied to the ablation expected area, or a visual indicator indicating a current flow on the recognized ablation electrode (215).
